# EUROPEAN PATENT APPLICATION

(11) **EP 1 352 907 A1**
(43) Date of publication of application: **15.10.2003**
(21) Application number: 01272276.5
(22) Date of filing: 21.12.2001
(51) Int. Cl.: C07D 493/22, C07D 311/16, A61K 31/366

(54) **PROCESSES FOR PREPARING CALANOLIDE A AND INTERMEDIATES THEREOF**

(30) Priority: 22.12.2000 JP 2000391248
(71) Applicant: Kuraray Co., Ltd., Kurashiki-City, Okayama 710-8622 (JP); SAGAMI CHEMICAL RESEARCH CENTER, Ayase-shi, Kanagawa 252-1193 (JP)
(72) Inventor: TERASHIMA, Shiro, Tokyo 156-0052 (JP); KOYAKUMARU, Kenichi, c/o KURARAY CO., LTD., Kurashiki-shi, Okayama 710-0801 (JP)
(74) Representative: von Kreisler, Alek, Dipl.-Chem.
(86) International application number: JP0111229
(87) International publication number: WO02051847

(57) **Abstract**

The present invention provides a production method of Calanolide A according to the following method

**Scheme 1**

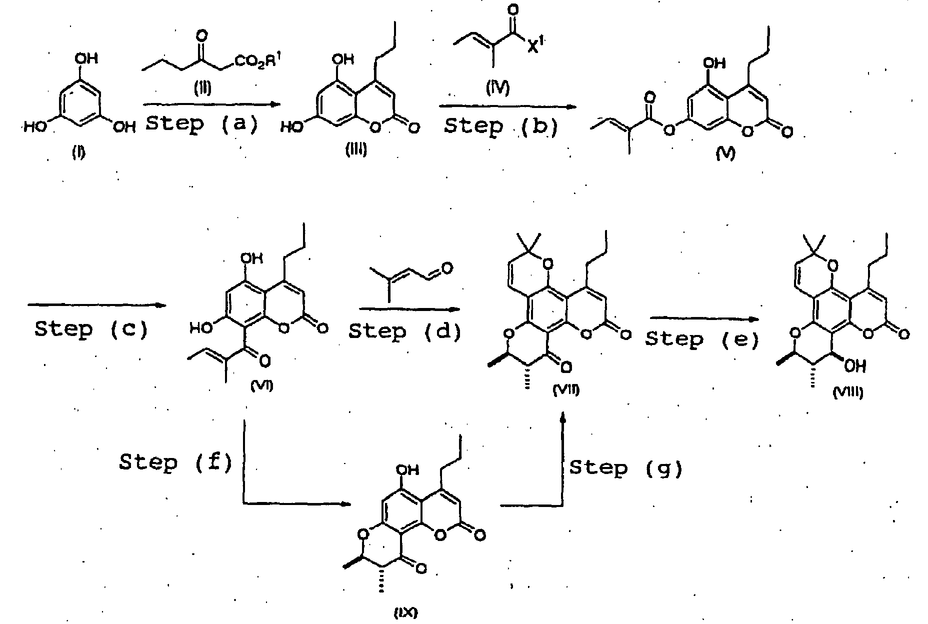

wherein each symbol is as defined in the specification, as a more convenient and industrially practical method for the synthesis of Calanolide A from an easily available starting material.

## Description

### Technical Field

The present invention relates to a production method of Calanolide A. Calanolide A produced by the present invention is useful as an HIV-1 reverse transcriptase inhibitor.

### Background Art

As a method for producing Calanolide A, the following methods are known.
(1) A method comprising a) acylating 5,7-dihydroxy-4-propylcoumarin in the presence of a Lewis acid to lead to 5,7-dihydroxy-8-propionyl-4-propylcoumarin, b) forming a chromene ring with 4,4-dimethoxy-2-methylbutane-2-ol, c) reacting the compound with acetaldehyde diethyl acetal to lead to 12-oxocalanolide A, and d) reducing the compound to give Calanolide A (see Patent No. 3043813).
(2) A method comprising a) acylating 5,7-dihydroxy-4-propylcoumarin with tigloyl chloride, b) cyclizing a tigloyl group in the presence of a base, c) converting the compound to (±)-12-oxocalanolide A by 3-chloro-3-methylbutyne and d) reduction to give Calanolide A (see Japanese Patent Application under PCT laid-open under kohyo No. hei-8-505146).
(3) A synthesis method of Calanolide A, comprising a) formylating 5,7-dihydroxy-4-propylcoumarin, b) forming a chromene ring with 3-chloro-3-methylbutyne, c) reacting with an optically active boron compound to obtain homoallylic alcohol, d) protecting with a silyl group, e) cyclization with a mercury compound accompanying oxidation, f) hydride reduction, g) deprotection of silyl group and h) inverting a hydroxyl group by Mitsunobu reaction (see WO96/26934).

However, the above-mentioned methods are problematic in that
in method (1), the yield of steps a) and c) is low, and the reaction of step b) requires several days,
in method (2), step c) requires use of an expensive reaction agent in excess, and the reaction time is comparatively long, in method (3), the number of steps is many, and reaction agents industrially difficult to use is employed, and the like.

### Disclosure of the Invention

It is therefore an object of the present invention to provide a more convenient and industrially practical method for the synthesis of Calanolide A from an easily available starting material.

According to the present invention, the above-mentioned object can be achieved by a method comprising
(a) a step of reacting phloroglucinol (I) 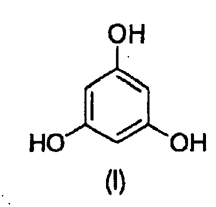 or a hydrate thereof, with a β-ketoester represented by the formula (II) 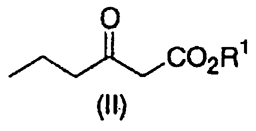 wherein R¹ is an alkyl group optionally having substituent(s), a cycloalkyl group optionally having substituent(s) or an aryl group optionally having substituent(s), in the presence of an acid catalyst to give 5,7-dihydroxy-4-propylcoumarin (III) 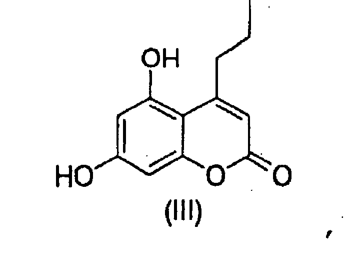
(b) a step of reacting 5,7-dihydroxy-4-propylcoumarin (III) with an acylating agent represented by the formula (IV) 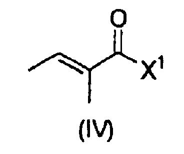 wherein X¹ is an alkoxyl group optionally having substituent(s), a cycloalkyloxy group optionally having substituent(s), an aryloxy group optionally having substituent(s), an acyloxy group optionally having substituent(s), an alkoxycarbonyloxy group optionally having substituent(s), a substituted amino group, a substituted sulfonyloxy group, a substituted phosphonyloxy group or a halogen atom to give 5-hydroxy-7-{(2-methyl)-2-butenoyloxy}-4-propylcoumarin (V) 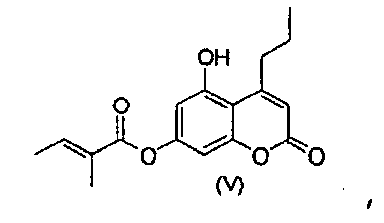
(c) a step of subjecting 5-hydroxy-7-{(2-methyl)-2-butenoyloxy}-4-propylcoumarin (V) to an intramolecular rearrangement reaction to give 5, 7-dihydroxy-8-{(2-methyl)-2-butenoyl}-4-propylcoumarin (VI) 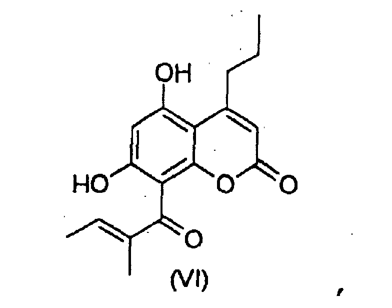
(d) a step of reacting 5,7-dihydroxy-8-{(2-methyl)-2-butenoyl}-4-propylcoumarin (VI) with 3-methyl-2-butenal to give 12-oxocalanolide A (VII) 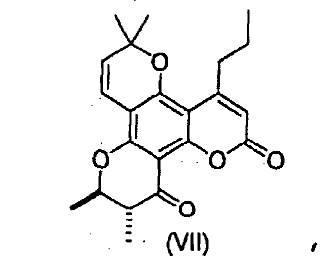 and
(e) a step of reducing 12-oxocalanolide A (VII) to give Calanolide A (VIII)

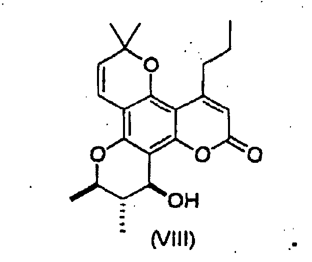

In addition, the object of the present invention can be also achieved by a method comprising
(a) reacting phloroglucinol (I) 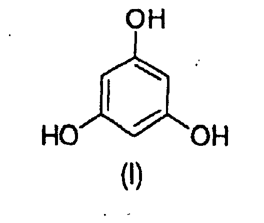 or a hydrate thereof, with a β-ketoser represented by the formula (II) 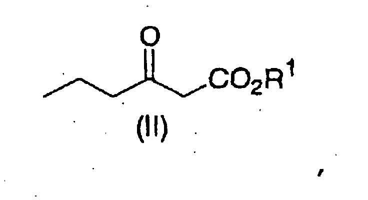 wherein R¹ is an alkyl group optionally having substituent(s), a cycloalkyl group optionally having substituent(s) or an aryl group optionally having substituent(s), in the presence of an acid catalyst to give 5,7-dihydroxy-4-propylcoumarin (III) 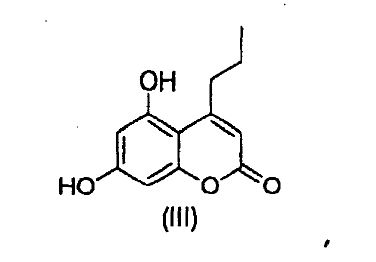
(h) a step of reacting 5,7-dihydroxy-4-propylcoumarin (III) with an acylating agent represented by the formula (X) 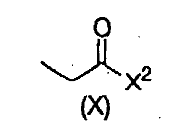 wherein X² is an alkoxyl group optionally having substituent(s), a cycloalkyloxy group optionally having substituent(s), an aryloxy group optionally having substituent(s), an acyloxy group optionally having substituent(s), an alkoxycarbonyloxy group optionally having substituent(s), a substituted amino group, a substituted sulfonyloxy group, a substituted phosphonyloxy group or a halogen atom, to give 5,7-dihydroxy-8-propionyl-4-propylcoumarin (XI) 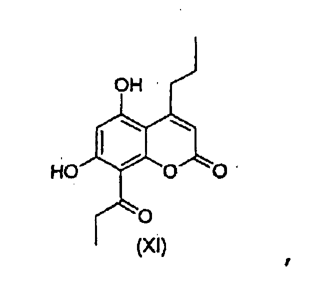
(i) a step of reacting 5,7-dihydroxy-8-propionyl-4-propylcoumarin (XI) with 3-methyl-2-butenal to give 5-hydroxy-2,2-dimethyl-6-propionyl-10-propyl-2H,8H-benzo[1,2-b:3,4-b']dipyran-8-one (XII) 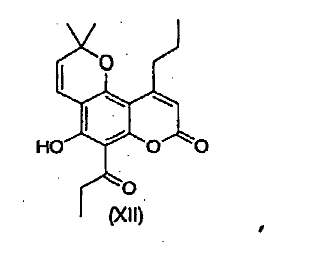
(j) a step of reacting 5-hydroxy-2,2-dimethyl-6-propionyl-10-propyl-2H,8H-benzo[1,2-b:3,4-b']-dipyran-8-one (XII) with an acetal of acetaldehyde represented by the formula (XIII) 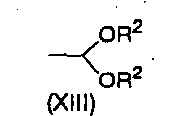 wherein two R² conveniently having the same superscript may be the same group or different groups, an alkyl group optionally having substituent(s), a cycloalkyl group optionally having substituent(s) or an aryl group optionally having substituent(s), or two R² may be joined to form a ring structure, in the presence of an acid catalyst to give 12-oxocalanolide A (VII) 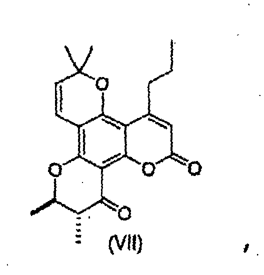 and
(e) a step of reducing 12-oxocalanolide A (VII) to give Calanolide A (VIII)

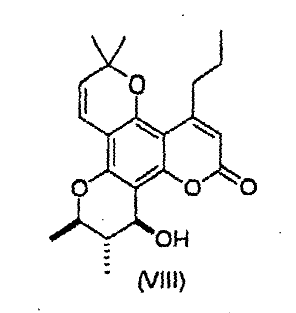

In the present specification, all the configurations of the structural formulas of the compounds having an asymmetric carbon atom show relative configurations, and said compound in the present invention means an optically active form or a racemate thereof unless particularly specified.

The preferable method for producing Calanolide A (VIII) according to the method of the present invention is shown in Scheme 1.

### Scheme 1

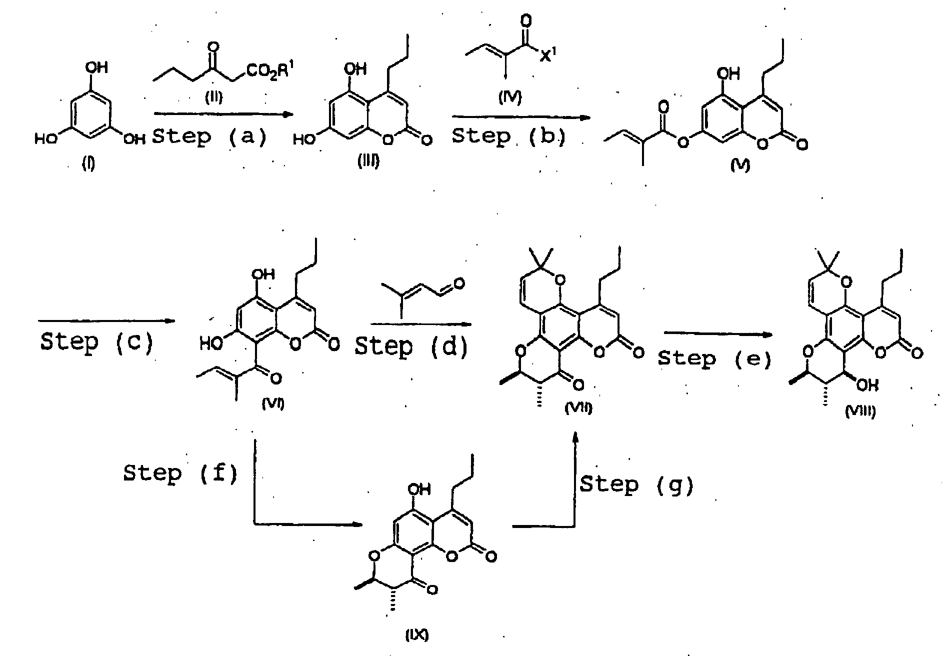

### Step (a): Step of obtaining 5, 7-dihydroxy-4-propylcoumarin (III) by reacting phloroglucinol (I) or a hydrate thereof with β-ketoester of the formula (II) in the presence of an acid catalyst

As the alkyl group represented by R¹ in the formula (II), a linear or branched alkyl group having 1 to 10 carbon atoms is preferable. For example, a methyl group, an ethyl group, a propyl group, an isopropyl group, a butyl group, an isobutyl group, a tert-butyl group and the like can be mentioned. As the cycloalkyl group represented by R¹, a cycloalkyl group having 3 to 8 carbon atoms is preferable. For example, a cyclopropyl group, a cyclopentyl group, a cyclohexyl group and the like can be mentioned. As the aryl group represented by R¹, for example, a phenyl group, a naphthyl group and the like can be mentioned. The above-mentioned alkyl group, cycloalkyl group and aryl group may have a substituent and as such substituent, for example, a halogen atom such as a chlorine atom, a bromine atom, an iodine atom and a fluorine atom; a nitro group; a linear or branched haloalkyl group preferably having 1 to to carbon atoms (wherein the halogen is as mentioned above) such as a trifluoromethyl group and the like; a linear or branched alkoxyl group preferably having 1 to 10 carbon atoms such as a methoxy group and the like; a linear or branched alkylcarbonyloxy group preferably having 2 to 11 carbon atoms such as an acetoxy group and the like; and the like can be mentioned.

Of the above-mentioned R¹, an ethyl group and a methyl group are preferable, and an ethyl group is particularly preferable.

Specific examples of β-ketoester of the formula (II) include methyl butyrylacetate, ethyl butyrylacetate, propyl. butyrylacetate, isopropyl butyrylacetate, butyl butyrylacetate, isobutyl butyrylacetate, tert-butyl butyrylacetate, cyclohexyl butyrylacetate, phenyl butyrylacetate, naphthyl butyrylacetate and the like. Preferred are ethyl butyrylacetate and methyl butyrylacetate, and particularly preferred is ethyl butyrylacetate. The amount of the β-ketoester to be used is preferably 0.3 - 3.0 equivalents, more preferably 0.8 - 1.2 equivalents, relative to phloroglucinol (I).

This reaction is carried out in the presence of an acid catalyst. As the acid catalyst, for example, sulfuric acid, hydrochloric acid, nitric acid, acetic acid, trifluoroacetic 'acid, methanesulfonic acid, benzenesulfonic acid, p-toluenesulfonic acid, trifluoromethanesulfonic acid and the like can be mentioned. Preferred are sulfuric acid, methanesulfonic acid and trifluoromethanesulfonic acid, and sulfuric acid is particularly preferable. These can be used alone or in combination of two or more kinds thereof. The amount of the acid catalyst to be used is preferably 0.1 - 10 equivalents, more preferably 1.0 - 3.0 equivalents, relative to phloroglucinol (I).

This reaction is preferably carried out by dropwise addition of an acid to a mixture of phloroglucinol (I) and β-ketoester of the formula (II), during which the temperature of the reaction mixture is preferably controlled to the range of 20°C - 120°C, particularly 60°C - 90°C, then preferably heated to a temperature within the range of 20°C - 120°C, more preferably 60°C - 90°C; untill the completion of the reaction.

A suitable organic solvent can be used for this reaction as long as it does not inhibit the reaction. As such organic solvent, for example, alcohol such as methanol, ethanol and 'the like; halogenated hydrocarbon such as dichloromethane, chloroform and the like; aromatic hydrocarbon such as benzene, toluene and the like; and the like can be mentioned. These can be used alone or in combination of two or more kinds thereof.

For isolation of the resulting product, a method generally used for isolating organic compounds can be used. For example, after pouring a reaction mixture into water, the mixture is extracted with an organic solvent such as ethyl acetate, dichloromethane, chloroform, tetrahydrofuran and the like. After washing the organic layer with water, the solvent is evaporated, and the resulting product can be isolated by a method such as recrystallization, silica gel chromatography and the like.

### Step (b): Step of obtaining 5-hydroxy-7-{(2-methyl)-2-butenoyloxy}-4-propylcoumarin (V) by reacting 5,7-dihydroxy-4-propylcoumarin (III) with an acylating agent of the formula (IV)

As the alkoxyl group represented by X¹ in the formula (IV), a linear or branched alkoxyl group having 1 to 10 carbon atoms is preferable, which is exemplified by a methoxy group, an ethoxy group, a propyloxy group, an isopropyloxy group, a butoxy group and the like. As the cycloalkyloxy group represented by X¹, a cycloalkyloxy group having 3 to 8 carbon atoms is preferable, which is exemplified by a cyclopropyloxy group, a cyclopentyloxy group, a cyclohexyloxy group and the like. As the aryloxy group represented by X¹, for example, a phenoxy group, a naphthoxy group and the like can be mentioned. As the acyloxy group represented by X¹, for example, a linear or branched alkylcarbonyloxy group preferably having 2 to 11 carbon atoms such as an acetyloxy group, a propionyloxy group, a butyryloxy group and the like; an arylcarbonyloxy group such as a benzoyloxy group and the like can be mentioned. As the alkoxycarbonyloxy group represented by X¹, a linear or branched alkoxycarbonyloxy group having 2 to 11 carbon atoms such as a methoxycarbonyloxy group, an ethoxycarbonyloxy group, a propyloxycarbonyloxy group, a butoxycarbonyloxy group, an isopropoxycarbonyloxy group and the like can be mentioned.

The above-mentioned alkoxyl group, cycloalkyloxy group, aryloxy group, acyloxy group and alkoxycarbonyloxy group may have a substituent, and as such substituent, for example, a halogen atom such as chlorine atom, bromine atom, iodine atom and fluorine atom; a nitro group; a linear or branched haloalkyl group (said halogen is as mentioned above) preferably having 1 to 10 carbon atoms such as trifluoromethyl group and the like; a linear or branched alkoxyl group preferably having 1 to 10 carbon atoms such as methoxy group and the like; a linear or branched alkylcarbonyloxy group preferably having 2 to 11 carbon atoms such as acetoxy group and the like; and the like can be mentioned.

As the substituted amino group represented by X¹, for example, an N-alkyl-N-alkoxyamino group (wherein the number of carbons of both alkyl group and alkoxyl group is preferably 1 - 10) such as an N-methyl-N-methoxyamino group, an N-methyl-N-ethoxyamino group and the like can be mentioned. As the substituted sulfonyloxy group represented by X¹, for example, a linear or branched alkanesulfonyloxy group preferably having 1 to 10 carbon atoms, such as a methanesulfonyloxy group and the like; an arylsulfonyloxy group such as a benzenesulfonyloxy group, a toluenesulfonyloxy group and the like; and the like can be mentioned. As the substituted phosphonyloxy group represented by X¹, a linear or branched dialkylphosphonyloxy group having 1 to 10 carbon atoms is preferable, such as a dimethylphosphonyloxy group, a diethylphosphonyloxy group and the like. As the halogen atom represented by X¹, a fluorine atom, a chlorine atom, a bromine atom and an iodine atom can be mentioned.

Of the above-mentioned X¹, a chlorine atom is preferable.

Specific examples of the acylating agent of the formula (IV) include methyl tiglate, ethyl tiglate, propyl tiglate, isopropyl tiglate, butyl tiglate, cyclohexyl tiglate, phenyl tiglate, naphthyl tiglate, acetic tiglic anhydride, propionic tiglic anhydride, benzoic tiglic anhydride, methoxycarbonic tiglic anhydride, N-methyl-N-methoxytiglic amide, methanesulfonic tiglic anhydride, tiglic p-toluenesulfonic anhydride, dimethoxyphosphoric tiglic anhydride, tigloyl fluoride, tigloyl chloride, tigloyl bromide, tigloyl iodide and the like, with preference given to tigloyl chloride. The amount of the acylating agent to be used is preferably 0.3-3.0 equivalents, more preferably 0.8 - 1.2, equivalents relative to 5,7-dihydroxy-4-propylcoumarin (III).

This reaction can be carried out in the presence of a Lewis acid catalyst. As the Lewis acid catalyst, for example, AlCl₃, ZnCl₂, TiCl₄, BF₃, SnCl₄, POCl₃ and the like can be mentioned, with preference given to AlCl₃. These can be used alone or in combination of two or more kinds thereof. The amount of the Lewis acid to be used is preferably 0.5 - 10 equivalents, more preferably 1.0 - 3.0 equivalents, relative to 5,7-dihydroxy-4-propylcoumarin (III).

For this reaction, a suitable organic solvent can be used as long as the reaction is not inhibited. As such organic solvent, for example, benzene, toluene, xylene, chlorobenzene, dichloromethane, chloroform, 1,2-dichloroethane and the like can be mentioned. These can be used alone or in combination of two or more kinds thereof.

This reaction is preferably carried out by dropwise addition of a mixture of a Lewis acid and an acylating agent of the formula (IV) prepared separately to a mixture of 5,7-dihydroxy-4-propylcoumarin (III) and a solvent, during which the temperature of the reaction mixture is preferably controlled to the range of -10°c to 50°C, particularly 10°C to 30°C.

For isolation of the resulting product, a method generally used for isolation of organic compounds can be used. For example, after pouring a reaction mixture into water, the mixture is extracted with an organic solvent such as ethyl acetate, dichloromethane, chloroform, tetrahydrofuran and the like, and, after evaporating the solvent the resulting product can be isolated by a method such as recrystallization, silica gel chromatography and the like.

### Step (c): Step for obtaining 5,7-dihydroxy-8-{(2-methyl)-2-butenoyl}-4-propylcoumarin (VI) by subjecting 5-hydroxy-7-{(2-methyl)-2-butenoyloxy}-4-propylcoumarin (V) to intramolecular rearrangement reaction

This reaction can be carried out in the presence of a Lewis acid catalyst. As the Lewis acid catalyst, for example, AlCl₃, ZnCl₂, TiCl₄, BF₃, SnCl₄, POCl₃ and the like can be mentioned, with preference given to AlCl₃. These can be used alone or in combination of two or more kinds thereof. The amount of the Lewis acid to be used is preferably 0.5 - 10 equivalents, more preferably 1.0 - 3.0 equivalents, relative to 5-hydroxy-7-[(2-methyl) -2-butenoyloxy}-4-propylcoumarin (V).

For this reaction, a suitable organic solvent can be used as long as the reaction is not inhibited. As such organic solvent, for example, benzene, toluene, xylene, chlorobenzene, dichloromethane, chloroform, 1,2-dichloroethane and the like can be mentioned. These can be used alone or in combination of two or more kinds thereof.

The reaction is preferably carried out in the temperature range of 20°C - 120°C, particularly 60°C - 90°c.

For isolation of the resulting product, a method generally used for isolation of organic compounds can be used. For example, after pouring a reaction mixture into water, the mixture is extracted with an organic solvent such as ethyl acetate, dichloromethane, chloroform, tetrahydrofuran and the like, after which the solvent is evaporated, and the resulting product can be isolated by a method such as recrystallization, silica gel chromatography and the like.

Where necessary, step (c) can be sequentially carried out without isolation of the product obtained in the above-mentioned step (b). That is, in the reaction of 5,7-dihydroxy-4-propylcoumarin (III) and an acylating agent of the formula (IV), after confirmation of production of 5-hydroxy-7-{(2-methyl)-2-butenoyloxy}-4-propylcoumarin (V), the reaction temperature is preferably raised to 20°C - 120°C, more preferably 60°C - 90°C to allow an intramolecular rearrangement reaction, whereby 5,7-dihydroxy-8-{(2-methyl)-2-butenoyl}-4-propylcoumarin (VI) can be obtained.

### Step (d): Step of obtaining 12-oxocalanolide A (VII) by reacting 5,7-dihydroxy-8-{(2-methyl)-2-butenoyl}-4-propylcoumarin (VI) with 3-methyl-2-butenal

The amount of the 3-methyl-2-butenal to be used is preferably 0.5 - 10 equivalents, more preferably 1.0 - 5.0 equivalents, relative to 5,7-dihydroxy-8-{(2-methyl)-2-butenoyl}-4-propylcoumarin (VI).

This reaction can be carried out in the presence of an acid catalyst. As the acid catalyst, for example, sulfuric acid, hydrochloric acid, nitric acid, acetic acid, boric acid, phenylboric acid, trifluoroacetic acid, methanesulfonic acid, benzenesulfonic acid, p-toluenesulfonic acid, trifluoromethanesulfonic acid and the like can be mentioned, with preference given to p-toluenesulfonic acid and sulfuric acid. These can be used alone or in combination of two or more kinds thereof. The amount of the acid catalyst to be used is preferably 0.001 - 10 equivalents, more preferably 0.01 - 0.1 equivalent, relative to 5,7-dihydroxy-8-{(2-methyl)-2-butenoyl}-4-propylcoumarin (VI).

This reaction can be also carried out in the presence of a base catalyst. As the base catalyst, for example, tertiary amines such as pyridine, picoline, lutidine, 4-dimethylaminopyridine, N,N-dimethylaniline, N,N-diethylaniline, trimethylamine, triethylamine, diisopropylethylamine, trioctylamine, triethanolamine, N-methylmorpholine, 1,8-diazabicyclo[5.4.0]-7-undecene and the like; inorganic salts such as lithium hydrogen carbonate, sodium hydrogen carbonate, potassium hydrogen carbonate, lithium carbonate, sodium carbonate, potassium carbonate and the like; and the like can be mentioned, with preference given to tertiary amines. These can be used alone or in combination of two or more kinds thereof. The amount of the base catalyst to be used is preferably 0.01 - 100 equivalents, more preferably 0.1 - 10 equivalents, relative to 5,7-dihydroxy-8-{(2-methyl)-2-butenoyl}-4-propylcoumarin (VI).

For this reaction, a suitable organic solvent can be used as long as the reaction is not inhibited. As such organic solvent, for example, benzene, toluene, xylene, chlorobenzene, chloroform, 1,2-dichloroethane, tetrahydrofuran, 1,2-dimethoxyethane, diisopropyl ether, tert-butylmethyl ether, methanol, ethanol, n-propanol, isopropanol, butanol, ethyl acetate, butyl acetate, N,N-dimethylformamide, dimethyl sulfoxide and the like can be mentioned. These can be used alone or in combination of two or more kinds thereof.

The reaction is preferably carried out in a range of 50°C - 150°C, particularly 70°C - 120°C.

For isolation of the resulting product, a method generally used for isolation of organic compounds can be used. For example, after pouring a reaction mixture into water, the mixture is, extracted with a suitable organic solvent such as ethyl acetate, dichloromethane, chloroform, tetrahydrofuran and the like, after which the solvent is evaporated, and the resulting product can be isolated by a method such as recrystallization, silica gel chromatography and the like.

More preferably, a worked-up crude product is recrystallized from a suitable solvent, and 12-oxocalanolide A (VII) alone can be selectively precipitated from a mixture of 12-oxocalanolide A (VII) and a diastereomer thereof: 10,11-cis-dihydro-6,6,10,11-tetramethyl-4-propyl-2H,6H,12H-benzo(1,2-b:3,4-b':5,6-b'']tripyran-2,12-dione (VII') 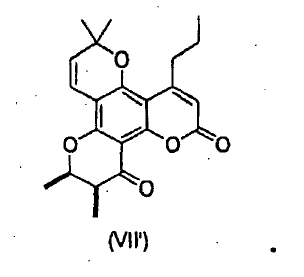 As the solvent usable for such recrystallization, for example, benzene, toluene, xylene, chlorobenzene, chloroform, 1,2-dichloroethane, tetrahydrofuran, 1,2-dimethoxyethane, diisopropyl ether, tert-butylmethyl ether, methanol, ethanol, n-propanol, isopropanol, butanol, ethylacetate, butyl acetate, N,N-dimethylformamide, dimethyl sulfoxide and the like can be mentioned. These can be used alone or in combination of two or more kinds thereof.

In addition, 12-oxocalanolide A (VII) can be also obtained from 5,7-dihydroxy-8-{(2-methyl)-2-butenoyl}-4-propylcoumarin (VI) by the following steps (f) and (g).

First, 5,7-dihydroxy-8-{(2-methyl)-2-butenoyl}-4-propylcoumarin (VI) is subjected to an intramolecular cyclization reaction to give 2,3-trans-dihydro-6-hydroxy-2,3-dimethyl-7-propyl-1H,9H-benzo[1,2-b:3,4-b']dipyran-1,9-dione (IX)

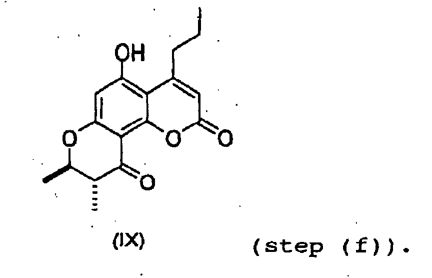

This reaction can be carried out in the presence of an acid catalyst. As the acid catalyst, for example, sulfuric acid, hydrochloric acid, nitric acid, acetic acid, boric acid, phenylboric acid, trifluoroacetic acid, methanesulfonic acid, benzenesulfonic acid, p-toluenesulfonic acid, trifluoromethanesulfonic acid and the like can be mentioned, with preference given to sulfuric acid and p-toluenesulfonic acid. These can be used alone or in combination of two or more kinds thereof. The amount of the acid catalyst to be used is preferably 0.001 - 10 equivalents, more preferably 0.01-0.1 equivalent, relative to 5,7-dihydroxy-8-{(2-methyl)-2-butenoyl}-4-propylcoumarin (VI).

This reaction can be also carried out in the presence of a base catalyst. As the base catalyst, for example, tertiary amines such as pyridine, picoline, lutidine, 4-dimethylaminopyridine, N,N-dimethylaniline, N,N-diethylaniline, trimethylamine, triethylamine, diisopropylethylamine, trioctylamine, triethanolamine , N-methylmorpholine, 1,8-diazabicyclo[5.4.0]-7-undecene and the like; inorganic salts such as lithium hydrogen carbonate, sodium hydrogen carbonate, potassium hydrogen carbonate, lithium carbonate, sodium carbonate, potassium carbonate and the like; and the like can be mentioned, with preference given to tertiary amines. These can be used alone or in combination of two or more kinds thereof. The amount of the base catalyst to be used is preferably 0.01 - 100 equivalents, more preferably 0.1 - 10 equivalents, relative to 5,7-dihydroxy-8-{(2-methyl)-2-butenoyl}-4-propylcoumarin (VI).

The reaction is preferably carried out in a range of 0°C - 120°C, particularly 40°C - 80°C.

This reaction can be also carried out without a catalyst. In this case, 5,7-dihydroxy-8-{(2-methyl)-2-butenoyl}-4-propylcoumarin (VI) is preferably heated to a range of 50°C - 140°C, particularly 70°C - 120°C.

For this reaction, a suitable organic solvent can be used as long as the reaction is not inhibited. As such organic solvent, for example, benzene, toluene, xylene, chlorobenzene, chloroform, 1,2-dichloroethane, tetrahydrofuran, 1,2-dimethoxyethane, diisopropyl ether, tert-butylmethyl ether, methanol, ethanol, n-propanol, isopropanol, butanol, ethyl acetate, butyl acetate, N,N-dimethylformamide, dimethyl sulfoxide and the like can be mentioned. These can be used alone or in combination of two or more kinds thereof.

For isolation of the resulting product, a method generally used for isolation of organic compounds can be used. For example, after pouring a reaction mixture into water, the mixture is extracted with a suitable organic solvent such as ethyl acetate, dichloromethane, chloroform, tetrahydrofuran and the like, after which the solvent is evaporated, and the product can be isolated by a method such as recrystallization, silica gel chromatography and the like.

Then, 2 ,3-trans-dihydro-6-hydroxy-2, 3-dimethyl-7-propyl-1H,9H-benzo[1,2-b:3,4-b']dipyran-1,9-dione (IX) is reacted with 3-methyl-2-butenal to give 12-oxocalanolide A (VII) (Step (g)).

The amount of 3-methyl-2-butenal to be used is preferably 0.5 - 10 equivalents, more preferably 1.0 - 5.0 equivalents, relative to 2, 3-trans-dihydro-6-hydroxy-2, 3-dimethyl-7-propyl-¹H, 9H-benzo[1, 2-b:3,4-b']dipyran-1,9-dione (IX).

This reaction can be carried out in the presence of an acid catalyst. As the acid catalyst, for example, sulfuric acid, hydrochloric acid, nitric acid, acetic acid, boric acid, phenylboric acid, trifluoroacetic acid, methanesulfonic acid, benzenesulfonic acid, p-toluenesulfonic acid, trifluoromethanesulfonic acid and the like can be mentioned, with preference given to sulfuric acid and p-toluenesulfonic acid. These can be used alone or in combination of two or more kinds thereof. The amount of the acid catalyst to be used is preferably 0.001 - 10 equivalents, more preferably 0.01 - 0.1 equivalent, relative to 2,3-trans-dihydro-6-hydroxy-2,3-dimethyl-7-propyl-1H,9H-benzo[1,2-b:3,4-b']dipyran-1,9-dione (IX).

This reaction can be also carried out in the presence of a base catalyst. As the base catalyst, for example, tertiary amines such as pyridine, picoline, lutidine, 4-dimethylaminopyridine, N, N-dimethylaniline, N,N-diethylaniline, trimethylamine, triethylamine, diisopropylethylamine, trioctylamine, triethanolamine , N-methylmorpholine, 1,8-diazabicyclo[5.4.0]-7-undecene and the like; lithium hydrogen carbonate, sodium hydrogen carbonate, potassium hydrogen carbonate, lithium carbonate, sodium carbonate, potassium carbonate and the like can be mentioned, with preference given to tertiary amines. These can be used alone or in combination of two or more kinds thereof. The amount of the base catalyst to be used is preferably 0.01-100 equivalents, more preferably 0.1 - 10 equivalents, relative to 2, 3-trans-dihydro-6-hydroxy-2,3-dimethyl-7-propyl-1H,9H-benzo[1,2-b:3,4-b']dipyran-1,9-dione (IX).

For this reaction, a suitable organic solvent can be used as long as the reaction is not inhibited. As such organic solvent, for example, benzene, toluene, xylene, chlorobenzene, chloroform, 1,2-dichloroethane, tetrahydrofuran, 1,2-dimethoxyethane, diisopropyl ether, tert-butylmethyl ether, methanol, ethanol, n-propanol, isopropanol, butanol, ethyl acetate, butyl acetate, N,N-dimethylformamide, dimethyl sulfoxide and the like can be mentioned. These can be used alone or in combination of two or more kinds thereof.

The reaction is preferably carried out in a range of 50°C - 150°C, particularly 70°C - 120°C.

The resulting product, 12-oxocalanolide A (VII), can be isolated in the same manner as in the above-mentioned Step (d).

Where necessary, Step (g) can be sequentially carried out without isolation of the product obtained in the above-mentioned Step (f). That is, 5,7-dihydroxy-8-{(2-methyl)-2-butenoyl}-4-propylcoumarin (VI) is subjected to intramolecular cyclization reaction and, after confirmation of production of 2, 3-trans-dihydro-6-hydroxy-2, 3-dimethyl-7-propyl-1H, 9H-benzo[1,2-b:3,4-b']dipyran-1,9-dione (IX), 3-methyl-2-butenal is added to allow reaction therewith, whereby 12-oxocalanolide A (VII) can be obtained.

### Step (e): Step for obtaining Calanolide A (VIII) by reduction of 12-oxocalanolide A (VII)

As the reducing agent to be used, for example, lithium aluminum hydride, lithium bis(2-methoxyethoxy)aluminum hydride, lithium tri-tert-butoxyaluminum hydride, sodium borohydride, lithium borohydride and the like can be mentioned, with preference given to lithium tri-tert-butoxyaluminum hydride. These can be used alone or in combination of two or more kinds thereof. The amount of the reducing agent to be used on conversion to hydride is preferably 0.5 - 10 equivalents, more preferably 1.0 - 3.0 equivalents, relative to 12-oxocalanolide A (VII).

A suitable organic solvent can be used for the reaction as long as the reaction is not inhibited., As such organic solvent, for example, benzene, toluene, xylene, tetrahydrofuran, 1,2-dimethoxyethane, diisopropyl ether, tert-butylmethyl ether and the like can be mentioned. These can be used alone or in combination of two or more kinds thereof.

The reaction is preferably carried out in a range of -50°C to 80°C, particularly -30°C to 40°C.

For isolation of the resulting product, a method generally used for isolation of organic compounds can be used. For example, a reaction mixture is poured into water and the mixture is extracted with a suitable organic solvent such as ethyl acetate, dichloromethane, chloroform, tetrahydrofuran and the like, and, after evaporating the solvent, the resulting product can be isolated by a method such as recrystallization, silica gel chromatography and the like.

More preferably, a worked-up crude product is recrystallized from a suitable solvent and Calanolide A (VIII) alone can be selectively precipitated from a mixture of Calanolide A (VIII) and a diastereomer thereof: Calanolide B (VIII') 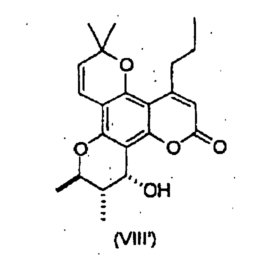 As the solvent usable for such recrystallization, for example, benzene, toluene, xylene, chlorobenzene, chloroform, 1,2-dichloroethane, tetrahydrofuran, 1,2-dimethoxyethane, diisopropyl ether, tert-butylmethyl ether, methanol, ethanol, n-propanol, isopropanol, butanol, ethyl acetate, butyl acetate, N,N-dimethylformamide, dimethyl sulfoxide and the like can be mentioned. These can be used alone or in combination of two or more kinds thereof.

Another preferable method for producing Calanolide A (VIII) according to the present invention is shown in Scheme 2.

### Scheme 2

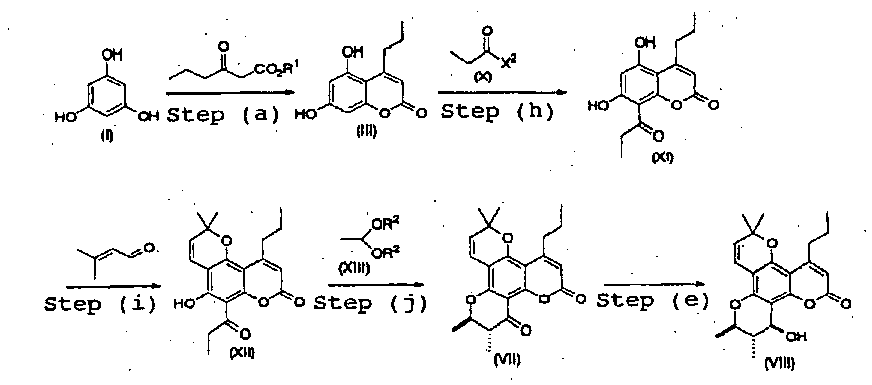

### Step (a): Step of obtaining 5,7-dihydroxy-4-propylcoumarin (III) by reacting phloroglucinol (I) or a hydrate thereof with β-ketoester of the formula (II) in the presence of an acid catalyst

This Step (a) is the same as Step (a) in the above-mentioned Scheme 1, and the kind and amount of the β-ketoester of the formula (II), the acid catalyst and the solvent to be used, as well as reaction conditions and the like are as defined above for Step (a).

### Step (h): Step of obtaining 5,7-dihydroxy-8-propionyl-4-propylcoumarin (XI) by reacting 5,7-dihydroxy-4-propylcoumarin (III) with an acylating agent of the formula (X)

As the alkoxyl group represented by X² in the formula (X), a linear or branched alkoxyl group having 1 to 10 carbon atoms is preferable, such as a methoxy group, an ethoxy group, a propyloxy group, an isopropyloxy group, a butoxy group and the like. As the cycloalkyloxy group represented by X², a cycloalkyloxy group having 3 to 8 carbon atoms is preferable, such as a cyclopropyloxy group, a cyclopentyloxy group, a cyclohexyloxy group and the like. As the aryloxy group represented by X², for example, a phenoxy group, a naphthoxy group and the like can be mentioned. As the acyloxy group represented by X², for example, a linear or branched alkylcarbonyloxy group preferably having 2 to 11 carbon atoms such as an acetyloxy group, a propionyloxy group, a butyryloxy group and the like; an arylcarbonyloxy group such as a benzoyloxy group and the like can be mentioned. As the alkoxycarbonyloxy group represented by X², a linear or branched alkoxycarbonyloxy group having 2 to 11 carbon atoms is preferable, such as a methoxycarbonyloxy group, an ethoxycarbonyloxy group, a propyloxycarbonyloxy group, a butoxycarbonyloxy group, an isopropoxycarbonyloxy group and the like.

The above-mentioned alkoxyl group, cycloalkyloxy group, aryloxy group, acyloxy group and alkoxycarbonyloxy group may have a substituent, and as such substituent, for example, a halogen atom such as chlorine atom, bromine atom, iodine atom and fluorine atom; a nitro group and the like can be mentioned.

As the substituted amino group represented by X², for example, an N-alkyl-N-alkoxyamino group (both alkyl group and alkoxyl group preferably have 1 to 10 carbon atoms) such as a N-methyl-N-methoxyamino group, a N-methyl-N-ethoxyamino group and the like can be mentioned.' As the substituted sulfonyloxy group represented by X², for example, a linear or branched alkanesulfonyloxy group preferably having 1 to 10 carbon atoms such as a methanesulfonyloxy group and the like; an arylsulfonyloxy group such as a benzenesulfonyloxy group, a toluenesulfonyloxy group and the like; and the like can be mentioned. As the substituted phosphonyloxy group represented by X², a linear or branched dialkylphosphonyloxy group having 1 to 10 carbon atoms is preferable, such as a dimethylphosphonyloxy group,' a diethylphosphonyloxy group and the like. As the halogen atom represented by X², a fluorine atom, a chlorine atom, a bromine atom and an iodine atom can be mentioned.

Of the above-mentioned X², a propionyloxy group is preferable.

Specific examples of the acylating agent of the formula (X) include methyl propionate, ethyl propionate, propyl propionate, isopropyl propionate, butyl propionate, methylcyclohexyl propionate, phenyl propionate, naphthyl propionate, acetic propionic anhydride, propionic acid anhydride, benzoic propionic anhydride, methoxycarbonic propionic anhydride, N-methyl-N-methoxy propionic amide, methanesulfonic propionic anhydride, p-toluenesulfonic propionic anhydride, dimethoxyphosphoric propionic anhydride and the like, with preference given to propionic acid anhydride. The amount of the acylating agent to be used is preferably 0.3 - 3.0 equivalents, more preferably 0.8 - 1.2 equivalents, relative to 5,7-dihydroxy-4-propylcoumarin (III).

This reaction can be carried out in the presence of a Lewis acid catalyst. As the Lewis acid catalyst, for example, AlCl₃, ZnCl₂, TiCl₄, BF₃, SnCl₄, POCl₃ and the like can be mentioned, with preference given to AlCl₃. These can be used alone or in combination of two or more kinds thereof. The amount of the Lewis acid to be used is preferably 0.5 - 10 equivalents, more preferably 1.0 - 3.0 equivalents, relative to 5,7-dihydroxy-4-propylcoumarin (III).

For this reaction, a suitable organic solvent can be used as long as the reaction is not inhibited. As such organic solvent, for example, benzene, toluene, xylene, chlorobenzene, dichloromethane, chloroform, 1,2-dichloroethane and the like can be mentioned. These can be used alone or in combination of two or more kinds thereof.

The reaction is preferably carried out in a range of 20°C -120°C, particularly 60°C - 90°C.

For isolation of the resulting product, a method generally used for isolation of organic compounds can be used. For example, after pouring a reaction mixture into water, the mixture is extracted with an organic solvent such as ethyl acetate, dichloromethane, chloroform, tetrahydrofuran and the like, and, after which the solvent is evaporated, the resulting product can be isolated by a method such as recrystallization, silica gel chromatography and the like.

### Step (i): Step of obtaining 5-hydroxy-2,2-dimethyl-6-propionyl-10-propyl-2H,8H-benzo[1,2-b:3,4--b']dipyran-8-one (XII) by reacting 5,7-dihydroxy-8-propionyl-4-propylcoumarin (XI) with 3-methyl-2-butenal

The amount of the 3-methyl-2-butenal to be used is preferably 0.5 - 10 equivalents, more preferably 1.0 - 5.0 equivalents, relative to 5,7-dihydroxy-8-propionyl-4-propylcoumarin (XI).

This reaction can be carried out in the presence of an acid catalyst. As the acid catalyst, for example, sulfuric acid, hydrochloric acid, nitric acid, acetic acid, boric acid, phenylboric acid, trifluoroacetic acid, methanesulfonic acid, benzenesulfonic acid, p-toluenesulfonic acid, trifluoromethanesulfonic acid and the like can be mentioned, with preference given to p-toluenesulfonic acid and sulfuric acid. These can be used alone or in combination of two or more .kinds thereof. The amount of the acid catalyst to be used is preferably 0.001 - 10 equivalents, more preferably 0.01 - 0.1 equivalent, relative to 5,7-dihydroxy-8-propionyl-4-propylcoumarin (XI).

This reaction can be also carried out in the presence of a base catalyst. As the base catalyst, for example, tertiary amines such as pyridine, picoline; lutidine, 4-dimethylaminopyridine, N,N-dimethylaniline, N,N-diethylaniline, trimethylamine, triethylamine, diisopropylethylamine, trioctylamine, triethanolamine , N-methylmorpholine, 1,8-diazabicyclo[5.4.0]-7-undecene and the like; inorganic salts such as lithium hydrogen carbonate, sodium hydrogen carbonate, potassium hydrogen carbonate, lithium carbonate, sodium carbonate, potassium carbonate and the like; and the like can be mentioned, with preference given to tertiary amines. These can be used alone or in combination of two or more kinds thereof. The amount of the base catalyst to be used is preferably 0.01 - 100 equivalents, more preferably 0.1 - 10 equivalents, relative to 5,7-dihydroxy-8-propionyl-4-propylcoumarin (XI).

For this reaction, a suitable organic solvent can be used as long as the reaction is not inhibited. As such organic solvent, for example, benzene, toluene, xylene, chlorobenzene, chloroform, 1,2-dichloroethane, tetrahydrofuran, 1,2-dimethoxyethane, diisopropyl ether, tert-butylmethyl ether, methanol, ethanol, n-propanol, isopropanol, butanol, ethyl acetate, butyl acetate, N,N-dimethylformamide, dimethyl sulfoxide and the like can be mentioned. These can be used alone or in combination of two or more kinds thereof.

The reaction is preferably carried out in a range of 50°C - 150°C, particularly 70°C - 120°C.

For isolation of the resulting product, a method generally used for isolation of organic compounds can be used. For example, after pouring a reaction mixture into water, the mixture is extracted with a suitable organic solvent such as ethyl acetate, dichloromethane, chloroform, tetrahydrofuran and the like, and, after evaporating the solvent, the resulting product can be isolated by a method such as recrystallization, silica gel chromatography and the like.

### Step (j): Step of obtaining 12-oxocalanolide A (VII) by reacting 5-hydroxy-2,2-dimethyl-6-propionyl-10-propyl-2H,8H-benzo[1,2-b:3,4-b']dipyran-8-one (XII) with an acetal of acetaldehyde of the formula (XIII) in the presence of an acid catalyst

As the alkyl group represented by R² in the formula (XIII), a linear or branched alkyl group having 1 to 10 carbon atoms is preferable, such as a methyl group, an ethyl group, a propyl group, an isopropyl group, a butyl group, an isobutyl group, a tert-butyl group and the like. As the cycloalkyl group represented by R², a cycloalkyl group having 3 to 8 carbon atoms is preferable, such as a cyclopropyl group, a cyclopentyl group, a cyclohexyl group and the like. As the aryl group represented by R², for example, a phenyl group, a naphthyl group and the like can be mentioned. The above-mentioned alkyl group, cycloalkyl group and aryl group may have a substituent, and as such substituent, for example, a halogen atom such as chlorine atom, bromine atom, iodine atom and fluorine atom; a nitro group and the like can be mentioned.

In addition, two R² may be joined to form a ring structure and the ring structure may contain an oxygen atom. As the ring structure to be formed, for example, a 1,3-dioxolane ring, a 1,3-dioxane ring and the like can be mentioned.

of the above-mentioned R², an ethyl group and a methyl group are preferable, and an ethyl group is particularly preferable.

specific examples of the acetal of acetaldehyde of the formula (XIII) include acetaldehyde dimethyl acetal, acetaldehyde diethyl acetal, acetaldehyde dipropyl acetal, acetaldehyde diisopropyl acetal, acetaldehyde dibutyl acetal, acetaldehyde diisobutyl acetal, acetaldehyde di-tert-butyl acetal, acetaldehyde dicyclohexyl acetal, acetaldehyde diphenyl acetal, acetaldehyde dinaphthyl acetal, 2-methyl-1,3-dioxolane, 2-methyl-1,3-dioxane and the like. Preferred are acetaldehyde diethyl acetal and acetaldehyde dimethyl acetal, and particularly preferred is acetaldehyde diethyl acetal. The amount of the acetal to be used is preferably 0.3 - 3.0 equivalents, more preferably 0.8 - 1.2 equivalents, relative to 5-hydroxy-2,2-dimethyl-6-propionyl-10-propyl-2H, 8H-benzo[1,2-b:3,4-b']dipyran-8-one (XII).

This reaction is carried out in the presence of an acid catalyst. As the acid catalyst, for example, sulfuric acid, hydrochloric acid, nitric acid, acetic acid, trifluoroacetic acid, methanesulfonic acid, benzenesulfonic acid, p-toluenesulfonic acid, trifluoromethanesulfonic acid and the like can be mentioned, with preference given to trifluoroacetic acid. These can be used alone or in combination of two or more kinds thereof. The amount of the acid catalyst to be used is preferably 0.1 - 10 equivalents, more preferably 1.0 - 3.0 equivalents, relative to 5-hydroxy-2,2-dimethyl-6-propionyl-10-propyl-2H,8H-benzo[1,2-b:3,4-b']dipyran-8-one (XII).

For this reaction, a suitable organic solvent can be used as long as the reaction is not inhibited. As such organic solvent, for example, benzene, toluene, xylene, chlorobenzene, chloroform, 1,2-dichloroethane and the like can be mentioned. These can be used alone or in combination of two or more kinds thereof.

The reaction is preferably carried out in a range of 60°C - 140°C, particularly 140°C.

For isolation of the resulting product, a method generally used for isolation of organic compounds can be used. for example, after pouring the reaction mixture into water, the mixture is extracted with an organic solvent such as ethyl acetate, dichloromethane, chloroform, tetrahydrofuran and the like, and, after washing the organic layer with water, the solvent is evaporated and the resulting product can be isolated by a method such as recrystallization, silica gel chromatography and the like.

More preferably, a worked-up crude product is recrystallized from a suitable solvent, and 12-oxocalanolide A (VII) alone can be selectively precipitated from a mixture of 12-oxocalanolide A (VII) and a diastereomer thereof, 10, 11-cis-dihydro-6,6,10,11-tetramethyl-4-propyl-2H,6H,12H-benzo[1,2-b:3,4-b':5,6-b"]tripyran-2,12-dione (VII') 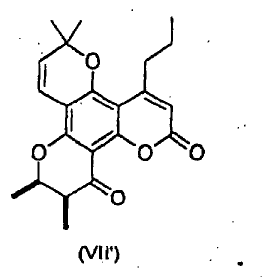 As the solvent usable for such recrystallization, for example, benzene, toluene, xylene, chlorobenzene, chloroform, 1,2-dichloroethane, tetrahydrofuran, 1,2-dimethoxyethane, diisopropyl ether, tert-butylmethyl ether, methanol, ethanol, n-propanol, isopropanol, butanol, ethyl acetate, butyl acetate, N,N-dimethylformamide, dimethyl sulfoxide and the like can be mentioned. These can be used alone or in combination of two or more kinds thereof.

### Step (e): Step of obtaining Calanolide A (VIII) by reduction of 12-oxocalanolide A (VII)

This Step (e) is the same as in Step (e) in the above-mentioned Scheme 1, and the kind and amount of the reducing ' agent and the solvent to be used, as well as the reaction conditions and the like are as defined above for Step (e).

The 10, 11-cis-dihydro-6, 6,10, 11,-tetramethyl-4-propyl-2H,6H,12H-benzo[4,2-b:3,4-b' :5,6-b'' ]tripyran-2,12-dione (VII') by-produced in the step for obtaining 12-oxocalanolide A (VII) can be converted to 12-oxocalanolide A (VII) by the following Steps (k) and (l).

First, 5-hydroxy-2, 2-dimethyl-6-{(2-methyl)-2-butenoyl}-10-propyl-2H,8H-benzo[1,2-b:3,4-b']dipyran-8-one (XIV) 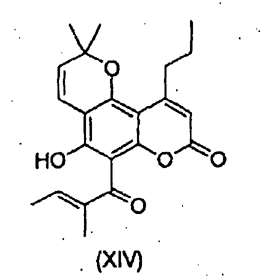 is obtained from 10, 11-cis-dihydro-6, 6,10, 11-tetramethyl-4-propyl-2H, 6H, 12H-benzo[1,2-b:3,4-b':5,6-b'']tripyran-2,12-dione (VII') (Step (k)).

In this reaction, a base is preferably used. As the base, for example, sodium carbonate, potassium carbonate, lithium hydroxide, sodium hydroxide, potassium hydroxide, sodium tert-butoxide, potassium tert-butoxide and the like can be mentioned, with preference given to sodium hydroxide and potassium hydroxide. These can be used alone or in combination of two or more kinds thereof. The amount of the base to be used is preferably 0.1 - 10 equivalents, more preferably 1.0-3.0 equivalents, relative to 10,11-cis-dihydro-6,6,10,11-tetramethyl-4-propyl-2H,6H,12H-benzo[1,2-b:3,4-b':5,6-b"]tripyran-2,12-dione (VII').

For this reaction, a suitable organic solvent can be used as long as the reaction is not inhibited. As such organic solvent, for example, benzene, toluene, xylene, chlorobenzene, chloroform, 1,2-dichloroethane, tetrahydrofuran, 1,2-dimethoxyethane, diisopropyl ether, tert-butylmethyl ether, methanol, ethanol, n-propanol, isopropanol, butanol, ethyl acetate, butyl acetate, N,N-dimethylformamide, dimethyl sulfoxide and the like can be mentioned. These can be used alone or in combination of two or more kinds thereof.

The reaction is preferably carried out in a range of - 20°C to 80°C, particularly to 40°C.

For isolation of the resulting product, a method generally used for isolation of organic compounds can be used. For example, after pouring a reaction mixture into water, the mixture is extracted with a suitable organic solvent such as ethyl acetate, dichloromethane, chloroform, tetrahydrofuran and the like, and, after evaporating the solvent, the resulting product can be isolated by a method such as recrystallization, silica gel chromatography and the like.

This Step is applicable to 10,11-dihydro-6,6,10,11-tetramethyl-4-propyl-2H,6H,12H-benzo[1,2-b:3,4-b':5,6-b"]tripyran-2,12-dione (VII") 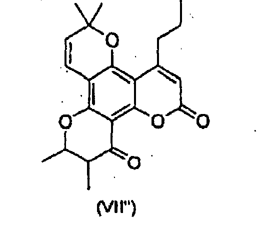 wherein the configurations of the 2-position and the 3-position are not important. In addition, application thereof to a mixture of 12-oxocalanolide A.(VII) and 10,11-cis-dihydro-6,6,10,11-tetramethyl-4-propyl-2H,6H,12H-benzo[1, 2-. b:3,4-b';5,6-b"]tripyran-2,12-dione (VII') wherein the ratio of 10, 11-cis-dihydro-6,6,10,11-tetramethyl-4-propyl-2H, 6H, 12H-benzo[1,2-b:3,4-b':5,6-b'']tripyran-2,12-dione (VII') is higher, is particularly effective.

Then,5-hydroxy-2,2-dimethyl.-6-{(2-methyl)-2 -butenoyl}-10-propyl-2H,8H-benzo[1,2-b:3,4-b']-dipyran-8-one (XIV) is subjected to a intramolecular cyclization reaction to give 12-oxocalanolide A (VII) (Step (1)).

This reaction can be carried out in the presence of a base catalyst. As the base catalyst, for example, tertiary amines such as pyridine, picoline, lutidine, 4-dimethylaminopyridine, N,N-dimethylaniline, N,N-diethylaniline, trimethylamine, triethylamine, diisopropylethylamine, trioctylamine, triethanolamine , N-methylmorpholine, 1,8-diazabicyclo[5.4 .0]-7-undecene and the like; inorganic salts such as lithium hydrogen carbonate, sodium hydrogen carbonate, potassium hydrogen carbonate, lithium carbonate, sodium carbonate, potassium carbonate and the like; and the like can be mentioned, with preference given to tertiary amines. These can be used alone or in combination of two or more kinds thereof. The amount of the base catalyst to be used is preferably 0.01 - 100 equivalents, more preferably 0.1 - 10 equivalents, relative to 5-hydroxy-2,2-dimethyl-6-{(2-methyl)-2-butenoyl}-10-propyl-2H,8H-benzo[1,2-b:3,4-b']dipyran-8-one (XIV).

This reaction can be also carried out in the presence of an acid catalyst. As the acid catalyst, for example, sulfuric acid, hydrochloric acid, nitric acid, acetic acid, boric acid, phenylboric acid, trifluoroacetic acid, methanesulfonic acid, benzenesulfonic acid, p-toluenesulfonic acid, trifluoromethanesulfonic acid and the like can be mentioned, with preference given to sulfuric acid and p-toluenesulfonic acid. These can be used alone or in combination of two or more kinds thereof. The amount of the acid catalyst to be used is. preferably 0.001.- 10 equivalents, more preferably 0.01 - 0.1 equivalent, relative to 5-hydroxy-2,2-dimethyl-6-{(2-methyl)-2-butenoyl}-10-propyl-2H,8H-benzo[1,2-b:3,4-b']dipyran-8-one (XIV).

The reaction is preferably carried out in a range of 0°C - 120°C, particularly 40°C - 80°C.

This reaction can be also carried out without a catalyst. In this case, 5-hydroxy-2, 2-dimethyl-6-{(2-methyl)-2-butenoyl}-10-propyl-2H,8H-benzo[1,2-b:3,4-b']dipyran-8-one (XIV) is preferably heated to 50°C - 140°C, particularly 70°c-120°C.

For this reaction, a suitable organic solvent can be used as long as the reaction is not inhibited. As such organic solvent, for example, benzene, toluene, xylene, chlorobenzene, chloroform, 1,2-dichloroethane tetrahydrofuran, 1,2-dimethoxyethane, diisopropyl ether, tert-butylmethyl ether, ethyl acetate, butyl acetate, N,N-dimethylformamide, dimethyl sulfoxide and the like can be mentioned. These can be used alone or in combination of two or more kinds thereof.

For isolation of the resulting product, a method generally used for isolation of organic compounds can be used. For example, after pouring a reaction mixture into water, the mixture is extracted with a suitable organic solvent such as ethyl acetate, dichloromethane, chloroform, tetrahydrofuran and the like, and, after evaporating the solvent, the resulting product can be isolated by a method such as recrystallization, silica gel chromatography and the like.

The 10,11-cis-dihydro-6,6,10,11-tetramethyl-4-propyl-2H,6H,12H-benzo[1,2-b:3,4-b':5,6-b"]tripyran-2,12-dione (VII') can be converted to 12-oxocalanolide A (VII) by a different method. That is, an acid is reacted with 10,11-cis-dihydro-6,6,10,11-tetramethyl-4-propyl-2H,6H,12H-benzo[1,2-b:3,4-b':5,6-b"]tripyran-2,12-dione (VII') to give 12-oxocalanolide A (VII) (Step (m)).

As the acid, for example, sulfuric acid, hydrochloric acid, nitric acid, acetic acid, boric acid, phenylboric acid, trifluoroacetic acid, methanesulfonic acid, benzenesulfonic acid, p-toluenesulfonic acid, trifluoromethanesulfonic acid and the like can be mentioned, with preference given to sulfuric acid and p-toluenesulfonic acid. These can be used alone or in combination of two or more kinds thereof. The amount of the acid to be used is preferably 0.001 - 10 equivalents, more preferably 0.01 - 0.1 equivalent, relative to 10,11-cis-dihydro-6,6,10,11-tetramethyl-4-propyl-2H,6H,12H-benzo[1,2-b:3,4-b':5,6-b"]tripyran-2,12-dione (VII').

The reaction is preferably carried out with heating to a range of 50°C - 140°C, particularly 70°c - 120°C.

For this reaction, a suitable organic solvent can be used as long as the reaction is not inhibited. As such organic solvent, for example, benzene, toluene, xylene, chlorobenzene, chloroform, 1,2-dichloroethane, tetrahydrofuran, 1,2-dimethoxyethane, diisopropyl ether, tert-butylmethyl ether, ethyl acetate, butyl acetate, N,N-dimethylformamide, dimethyl sulfoxide and the like can be mentioned. These can be used alone or in combination of two or more kinds thereof.

For isolation of the resulting product, a method generally used for isolation of organic compounds can be used. For example, after pouring a reaction mixture into water, the mixture is extracted with a suitable organic solvent such as ethyl acetate, dichloromethane, chloroform, tetrahydrofuran and the like, and, after evaporating the solvent, the' resulting product can be isolated by a method such as recrystallization, silica gel chromatography and the like.

When the ratio of 10,11-cis-dihydro-6,6,10,11-tetramethyl-4-propyl-2H,6H,12H-benzo[1,2-b:3,4-b':5,6-b"]tripyran-2,12-dione (VII') in the product obtained in the Step to obtain 12-oxocalanolide A (VII) is high, the above-mentioned Steps (k) and (1), or Step (m) can be applied. That is, a mixture of 12-oxocalanolide A (VII) and 10,11-cis-dihydro-6, 6,10,1 1-tetramethyl-4-propyl-2H, 6H, 12H-benzo [1,2-b;3,4-b':5,6-b'']tripyran-2,12-dione (VII'), which has a higher ratio of 10,11-cis-dihydro-6,6,10,11-tetramethyl-4-propyl-2H,6H,12H-benzo[1,2-b:3,4-b':5,6-b" ]tripyran-2,12-dione (VII') can be converted to a mixture of 12-oxocalanolide A (VII) and 10,11-cis-dihydro-6,6,10,11-tetramethyl-4-propyl-2H,6H,12H-benzo[1,2-b:3,4-b':5,6-b'']tripyran-2,12-dione (VII'), which has a higher ratio of 12-oxocalanolide A (VII). As a result, 12-oxocalanolide A (VII) can be selectively precipitated as mentioned above, and the efficiency of the Step to obtain 12-oxocalanolide A (VII) can be increased.

### Examples

The present invention is explained in detail in the following by referring to Examples. It is needless to say that the present invention is not limited to the following Examples.

### Example 1: 5,7-dihydroxy-4-propylcoumarin (III) [Step (a)]

Concentrated sulfuric acid (2.15 g, 21.9 mmol) was added to a suspension of phloroglucinol dihydrate (I) (3.0 g, 18.5 mmol) and ethyl butyrylacetate(3.23 g, 20.4 mmol). The reaction mixture became a homogeneous yellow solution with generation of heat and then solidified. After allowing the solid to stand at room temperature for 2 hrs., the obtained solid was thoroughly pulverized. The pulverizate was washed with water and the product was filtrated. Washing with water and filtration was repeated until the washing became pH 3. Thorough drying gave the title compound as a pale-yellow powder (3.92 g, yield 96%). mp; 227 - 229°C
¹H-NMR(DMSO); δ 0.95(t, 3H, J=7.3Hz), δ 1.58(sextet, 2H, J=7.4Hz), δ 2.85(t, 2H, J=7.5Hz), δ 5.83(s, 1H), δ 6,19(d, 1H, J=2.3Hz), δ 6.27(d, 1H, J=2.3Hz), δ 10.30 (s, 1H), δ 10.59 (s, 1H)
¹³C-NMR(DMSO); δ 13.82, δ 22.53, δ 37.24, δ 94.74, δ 99.17, δ 99.26, δ 101.37, δ 108.28, δ 108.32, δ 156.84, δ 157.42, δ 158.51, δ 160.18, δ 160.91
MS; 221(13.8, M+1), 220(100, M+), 205(37.0), 192(73.4), 177(30.0)
IR(KBr); 3204, 1649, 1622, 1591, 1561 cm-¹

### Example 2: 5-hydroxy-7-{(2-methyl)-2-butenoyloxy}-4-propylcoumarin (V) [Step (b)]

In a three-neck flask equipped with an efficient mechanical stirrer, a thermometer and a dropping funnel after nitrogen displacement were filled with 5,7-dihydroxy-4-propylcoumarin (III) (200 mg, 0.91 mmol) and 1,2-dichloroethane (1 ml), and the mixture was stirred at room temperature. Thereto was dropwise added a solution of tigloyl chloride (350 mg, 2.92 mmol) and aluminum chloride (360 mg, 2.72 mmol) in 1,2-dichloroethane (1 ml) at room temperature. The reaction mixture became a yellow solution while foaming vigorously. After the completion of the dropwise addition, the mixture was stirred at room temperature for 30 min. Water (3 ml) was added thereto and the mixture was extracted with ethyl acetate.The organic layers were collected, washed with saturated aqueous sodium hydrogen carbonate solution and dried over magnesium sulfate. The solvent was evaporated to give a pale-brown solid. This was washed with a mixture of ethyl acetate and hexane (1:1) and then with a mixture of ethyl acetate and hexane (1:4) to give a white solid (241 mg,yield 88%) .
mp; 153 - 154°C
¹H-NMR(CDCl₃); δ 0.94(t, 3H, J=7.3Hz), δ 1.49(sextet, 2H, J=7.4Hz), δ 1.93(d, 3H, J=7.1Hz), δ 1.97(s, 3H), δ 2.74(t, 2H, J=7.6Hz), δ 6.03(s, 1H), δ 6.39(d, 1H, J=2.3Hz), δ 6.64(d, 1H, J=2.3Hz), δ 7.21(q, 1H, J=7.0Hz), δ 7.36(s, 1H)
¹³C-NMR(CDCl₃); δ 12.12, δ 13.89, δ 14.91, δ 22.42, δ 37.84, δ 102.82, δ 105.99, δ 107.15, δ 112.56, δ 127.52, δ 142.04, δ 152-88, δ 155.61, δ 156.35, δ 158.10, δ 160.85, δ 167.13 MS(EI); 303(2.6, M+1), 302(13.3, M+), 220(2.8), 83(100), 55(31.6)
IR(KBr); 3107, 2969, 1730, 1680, 1609 cm⁻¹

### Example 3:5,7-dihydroxy-8-{(2-methyl)-2-butenoyl}-4-propylcoumarin (VI) [one pot step from Step (b) to Step (c)]

A three-neck reaction vessel equipped with an efficient mechanical stirrer, a thermometer, a reflux condenser and a dropping funnel, and an aqueous sodium hydroxide solution tank capable of ventilation from the reaction vessel were prepared and the whole atmosphere was displaced with argon. Into this reaction vessel were charged 5,7-dihydroxy-4-propylcoumarin (III) (10.0 g, 45.4 mmol) and 1,2-dichloroethane (30 ml) and the mixture was stirred at room temperature. Thereto was dropwise added a solution of tigloyl chloride (8.94 g, 75.4 mmol) and aluminum chloride (18.2 g, 136.2 mmol) in 1,2-dichloroethane (20 ml) at room temperature over 20 min. The reaction mixture became a yellow-brown solution while foaming vigorously. After the completion of the dropwise addition, the mixture was stirred at room temperature for 30 min. The mixture was subjected to TLC analysis and disappearance of the starting ,material and generation of 5-hydroxy-7-{(2-methyl)-2-butenoyloxy}-4-propylcoumarin (V) were confirmed. The internal temperature was raised to 50°c and the mixture was stirred for 30 min. The mixture was further stirred at an internal temperature of 70°C for 3.5 hrs. The hydrogen chloride generated during the stirring was flown to the aqueous sodium hydroxide solution tank for neutralization. Then, the reaction mixture was poured into water (200 ml) at a liquid temperature of not lower than 50°C.

The precipitated pale-brown solid was collected by filtration and washed with water (100 ml) and then a mixture of ethyl acetate and hexane (1:3) (40 ml). The solid product was dissolved in a mixture of ethyl acetate (200 ml) and ethanol (100 ml), and an insoluble material was filtered off with celite. The filtrate was concentrated to give a pale-brown solid (10.8 g, yield 79%).
mp; 213 - 215°C
¹H-NMR(CDCl₃/DMSO=10/1); δ 1.00(t, 3H, J=7.3Hz), δ 1.66(sextet, 2H, J=7.4Hz), δ 1.83(d, 3H, J=6.9Hz), δ 1.94(s, 3H), δ 2.92(t, 2H, J=7.5Hz), δ 5.87(s, 1H), δ 6.32(q, 1H, J=6.9Hz), δ 6.41(s, 1H), 10.41(brs, 1H), δ 10.78(brs, 1H)
¹³C-NMR(CDCl₃/DMSO=10/1); δ 11.94, δ 14.00, δ 14.60, δ 22.77, δ 38.19, δ 99.70, δ 102.47, δ 106.01, δ 109.28, δ 136.94, δ 137.86, δ 139.40, δ 155.39, δ 159.10, δ 159.90, δ 160.34, δ 161.69
MS(EI); 303(4.9, M+1), 302(24.3, M+), 288(20.8), 287(100), 259(47.8), 100(14.9), 55(10.1)
IR(KBr); 3299, 1694, 1622, 1588 cm⁻¹

### Example 4:(±)-12-oxocalanolide A (VII) [Step (d) in the presence of base catalyst]

Into a three-neck flask equipped with Soxhlet extractor (with molecular sieves 4A), a reflux condenser and a thermometer were charged 5,7-dihydroxy-8-{(2-methyl)-2-butenoyl}-4-propylcoumarin (VI) (10.8 g, 35.8 mmol), 3-methyl-2-butenal (7.0 ml, 72.6 mmol) and pyridine (30 ml), and the mixture was heated under reflux for 5.5 hrs. After the completion of heating, the mixture was cooled to room temperature and ethyl acetate (130 ml), ethanol (50 ml) and water (30 ml) were added thereto. Hydrochloric acid was added until the aqueous layer was acidified and the aqueous layer was removed. The organic layer was washed with water and dried over magnesium sulfate. A silica gel layer was filtered off and the solvent was evaporated to give a crude product (10.38 g, 79%). The product was recrystallized from ethyl acetate to give (±)-12-oxocalanolide A (VII) as colorless plates (3.38 g, yield 26%). The mother liquor was concentrated and purified by silica gel chromatography to give (±)-12-oxocalanolide A (VII) (2.34 g, yield 18%) and (±)-10,11-cis-dihydro-6,6,10,11-tetramethyl-4-propyl-2H,6H,12H-benzo[1,2-b:3,4-b':5,6-b'']tripyran-2,12-dione (VII') (2.57 g, yield 19%), which was a diastereomer thereof.

### (±)-12-oxocalanolide A (VII)

mp; 184 - 186°C
¹H-NMR(CDCl₃); δ 1.03(t, 3H, J=7.3Hz), δ 1.21(d, 3H, J=6.9Hz), δ 1.52(s, 3H), δ 1.53(d, 3H, J=6.4Hz), δ 1.55(s, 3H), δ 1.64(sextet, 2H, J=7.5Hz), δ 2.55(dq, 1H, J=6.3Hz, 11.1Hz), δ 2.86-2.89(m, 2H), δ 4.29(dq, 1H, J=6.3Hz, 11.1Hz), δ 5.60(d, 1H, J=10.1Hz), δ 6.03(s, 1H), δ 6.65(d, 1H, J=10.1Hz) ¹³C-NMR(CDCl₃); δ 10.47, δ 13.88, δ 19.59, δ 23.13, δ 27.97, δ 28.28, δ 38.72, δ 47.27, δ 79.21, δ 79.57, δ 103.51, δ 104.42, δ 105.46, δ 112.04, δ 115.82, δ 126.97, δ 155.51, δ 155.91, δ 157.04, δ 159.04, δ 159.69, δ 189.89
MS(EI); 369(9.4, M+1), 368(38.6, M+), 354(21.4), 353(100), 325(6.7), 312(6.7), 297(34.3), 269(12.3),
IR(KBr); 2967, 2934, 1740, 1684, 1557, 1200 cm⁻¹ (±)-10, 11-cis-dihydro-6, 6, 10, 11-tetramethyl-4-propyl-2H,6H,12H-benzo[1,2-b:3,4-b':5,6-b " ]tripyran-2,12-dione (VII')
mp; 101 - 103°C
¹H-NMR(CDCl₃); δ 1.03(t, 3H, J=7.3Hz), δ 1.16(d, 3H, J=7.2Hz), δ 1.42(d, 3H, J=6.6Hz), δ 1.54(s, 3H), δ 1.54(s, 3H), δ 1.64(sextet, 2H, J=7.5Hz), δ 2.68(dq, 1H, J=3.4Hz, 7.2Hz), δ 2.83-2.93(m, 2H), δ 4.70(dq, 1H, J=3.4Hz, 6.6Hz), δ 5.60(d, 1H, J=10.1Hz), δ 6.04(s, 1H), δ 6.64 (d, 1H, J=10.1Hz)
¹³C-NMR(CDCl₃); δ 9.18, δ 13.87, δ 16.00, δ 23.13, δ 28.12, δ 28.24, δ 38.73, δ 45.92, δ 77.20, δ 79.27, δ 102.90, δ 104.45, δ 105.51, δ 112.01, δ 115.83, δ 126.99, δ 155.76, δ 156.03, δ 157.13, δ 158.87, δ 159.78, δ 191.53
MS(EI); 369(9.3, M+1), 368(32.8, M+), 354(26.3), 353(100), 325(14.5), 297(36.9), 269(19.1)
IR(KBr); 2967, 2934, 1736, 1688, 1572, 1120 cm⁻¹

### Example 5:(±)-Calanolide A (VIII) [Step (e)]

Under an argon atmosphere, LiAlH(otsu)₃ (828 mg, 3.26 mmol) was added to a solution of (±)-12-oxocalanolide A (VII) (1.00 g, 2.71 mmol) cooled to -15°C in THF (15 ml). The temperature of the mixture was raised to room temperature over 2.5 hrs. and cooled to -6°C. LiAlH(OtBu)₃ (207 mg, 0.82 mmol) was added and the mixture was further stirred for 13 hrs. Water was added to the reaction mixture and hydrochloric acid was added to adjust the aqueous layer to pH 3. The layer was extracted with ethyl acetate. The organic layers were collected and dried over magnesium sulfate. The solvent was evaporated to give (±)-Calanolide A (VIII) and (±)-Calanolide B (VIII') as a diastereomer mixture (970 mg). By the analysis for ¹H-NMR, the (±)-calanolide A:(±)-Calanolide B ratio was 4.6:1.0. Therefrom the reaction yield of (±)-Calanolide A was calculated to be 80%. The diastereomer mixture of (±)-Calanolide A and (±)-Calanolide B was recrystallized twice from a mixture of ethyl acetate and isopropyl ether (1:14) to give pure (±)-Calanolide A as colorless crystals (403 mg). mp; 78 - 79°C
¹H-NMR(CDCl₃); δ 1.03(1, 3H, J=7.3Hz), δ 1.15(d, 3H, J=6.8Hz), δ 1.46-1.47(m, 6H), δ 1.51(s, 3H), δ 1.55(s, 3H), δ 1.66(sextet, 2H, J=7.4Hz), δ 1.89-1.97(m, 1H), δ 2.83-2.96(m, 2H), δ 3.80-3.89(br, 1H), δ 3.93(dq, 1H, J=6.4Hz, 8.9Hz), δ 4.71(d, 1H, J=7.7Hz), δ 5.54(d, 1H. J=10.0Hz), δ 5.95(s, 1H), δ 6.62(d, 1H, J=10.0Hz)
¹³C-NMR(CDCl₃); δ 14.00, δ 16.16, δ 18.97, δ 23.29, δ 27.42, δ28.05, δ 38.69, δ 40.53, δ 67.10, δ 77.29, δ 77.72, δ 104.11, δ 106.42, δ 106.51, δ 110.10, δ 116.56, δ 127.00, δ 151.18, δ 153.21, δ 154.52, δ 159.09, δ 160.77
MS(EI); 371(8.1, M+1), 370(28.2, M+), 356(26.1), 355(100), 337(21.9), 299(29.5)
IR(KBr); 3484, 3260, 2971, 1698, 1584 cm⁻¹

### Example 6:(±)-12-oxocalanolide A (VII) [Step (d) in the presence of acid catalyst]

5,7-Dihydroxy-8-{(2-methyl)2-butenoyl}-4-propylcoumarin (VI) (101 mg, 0.33 mmol), 3-methyl-2-butenal (90 mg, 1.07 mmol) and p-toluenesulfonic acid monohydrate (13 mg, 0.07 mmol) were dissolved in toluene (1 ml) and the mixture was heated at 110°C for 4 hr. Water was added to the reaction mixture, and the mixture was extracted with ethyl acetate. After drying over magnesium sulfate, the solvent was evaporated and the residue was purified by silica gel chromatography to give the title compound as a brown solid (12 mg, yield 10%).

### Example 7: (±)-2,3-trans-dihydro-6-hydroxy-2,3-dtmethyl-7-propyl-1H,9H-benzo[1,2-b:3,4-b']dipyran-1,9-dione (IX) [Step (f)]

5,7-Dihydroxy-8-{(2-methyl)-2-butenoyl}-4-propylcoumarin (VI) (1.0 g, 3.3 mmol) and triethylamine (0.2 ml 1.4 mmol) were dissolved in methanol (10 ml) and the mixture was heated at 60°C for 4.5 hrs. The reaction mixture was concentrated, and after dissolving in ethyl acetate, passed through a silica gel layer. The solvent was evaporated to give a brown solid (927 mg, 93%). This was subjected to ¹H-NMR measurement. As a result, it was found to be a mixture of diastereomers of the title compound and the corresponding cis form.and the production ratio was 1.00:0.65. Therefore, the yield of the title compound was calculated to be 56%. This crude product was purified by silica gel chromatography to give pure (±)-2,3-trans-dihydro-6-hydroxy-2,3-dimethyl-7-propyl-1H, 9H-benzo[1,2-b;3,4-b']dipyran-1,9-dione (IX) as a white solid. mp; 238 - 240°C
¹H-NMR(CDCl₃); δ 0.98(t, 3H, J=7.3Hz), δ 1.19(d, 3H, J=6.9Hz), δ 1.48(d, 3H, J=6.3Hz), δ 1.60(sextet, 2H, J=7.4Hz), δ 2.51(dq, 1H, J=6.9Hz, 11.1Hz), δ 2.88(tABq, 2H, J=7.5Hz, 13.9H2), δ 4.22(dq, 1H, J=6.3Hz, 11,.IHz), δ 6.01(s, 1H), δ 6.17(s, 1H)
¹³C-NMR(CDCl₃); δ 10.60, δ 13.91, δ 19.66, δ 22.71, δ 38.33, δ 47.36, δ 79.28; δ 99.24, δ 103.12, δ 104.35, δ 110.75, δ 156.42, δ 158.80, δ 160.85, δ 161.69, δ 164.39, δ 190.56
MS(EI); 303(10.4, M+1), 302(52.9, M+), 247(16.4), 246(100), 217(34.0), 203(17.3), 190(21.1)
IR(KBr); 3086, 2961, 1721, 1609, 1209 cm⁻¹

### Example 8: (±)-12-oxocalanolide A (VII) [step (g)]

Into a three-neck flask equipped with a soxhlet extractor (with molecular sieves 4A), a reflux condenser and a thermometer were charged (±)-2,3-trans-dihydro-6-hydroxy-2,3-dimethyl-7-propyl-1H,9H-benzo[1,2-b:3,4-b']dipyran-1,9-dione (IX) (50 mg, 0.17 mmol), 3-methyl-2-butenal (144 mg, 1.71 mmol) and pyridine (1 ml), and the mixture was heated at 120°C for 8 hrs. The reaction mixture was concentrated, purified by silica gel chromatography to give the ,title compound as a white solid (36 mg, yield 59%).

### Example 9: 5,7-dihydroxy-8-propionyl-4-propylcoumarin (XI) [Step (h)]

A three-neck reaction vessel equipped with an efficient mechanical stirrer, a thermometer, a reflux condenser and a dropping funnel was prepared and the whole atmosphere was displaced with argon. 5,7-Dihydroxy-4-propylcoumarin (III) (3.5 g, 15.9 mmol), aluminum chloride (2.12 g, 15.9 mmol) and 1,2-dichloroethane (15 ml) were charged in this reaction vessel and the mixture was stirred with heating at an internal temperature of 70 - 75°C. Thereto was dropwise added a separately prepared solution of propionic acid anhydrate (2.04 ml, 15.9 mmol) and aluminum chloride (4.12 g, 31.8 mmol) in 1,2-dichloroethane (10 ml) over about 2 hrs and the mixture was further heated at the same temperature for 1 hr. After cooling to room temperature, the reaction mixture was poured into 1N HC1 under ice-cooling with vigorous stirring. The mixture was extracted with a mixed solvent of ethyl acetate (60 ml) and ethanol (10 ml). After drying over magnesium sulfate, the residue was concentrated to give a pale-yellow solid. This was recrystallized from 1,4-dioxane (20 ml) to give white needles (1.41 g, yield 32%). mp; 248 - 249°C
¹H-NMR(DMSO); δ 0.95(t, 3H, J=7.3Hz), δ 1.08(d, 3H, J=7.2Hz), δ 1.58 (sextet, 2H, J=7.4HZ), δ 2.78(t, 2H, J=7.5Hz), δ 3.02(q, 2H, J=7.2Hz), δ 5.97(s, 1H), δ 6.31(s, 1H), δ 11.49(brs, 1H), δ 12.58(s, 1H)
¹³C-NMR(DMSO); δ 8.13, δ 13.81, δ 22.59, δ 37.28, δ 37.52, δ 99.09, δ 101:69, δ 106.17, δ 108.92, δ 155.99, δ 158.64, δ 158.86, δ 160.44, δ 162.73, δ 204.26
MS(EI); 277(7.2, M+1), 276(43.1, M+), 247(100), 219(21.0)
IR(KBr); 3248, 1693, 1626, 1593, 1398, 1211 cm⁻¹

### Example 10: 5-hydroxy-2,2-dimethyl-6-propionyl-10-propyl-2H,8H-benzo[1,2-b:3,4-b']dipyran-8-one (XII) [Step (i) in the presence of base catalyst]

5,7-Dihydroxy-8-propionyl-4-propylcoumarin (XI) (100 mg, 0.36 mmol), 3-methyl-2-butenal (150 mg, 1.78 mmol) and pyridine (0.3ml) were charged and the mixture was heated at 120°C for 5 hrs. After evaporation of pyridine, the residue. was purified by silica gel chromatography to give the title. compound as a pale-yellow solid (120 mg, yield 94%). mp; 110 - 111°C
¹H-NMR(CDCl₃); δ 1.05(t, 3H, J=7.3Hz), δ 1.23(d, 3H, J=7.1Hz), δ 1.53(s, 6H), δ 1.66(sextet, 2H, J=7.5Hz), δ 2.91(t, 2H, J=7.7Hz), δ 3.35(q, 2H, J=7.1Hz), δ 5.58(d, 1H, J=10.1Hz), δ 6.01(s, 1H), δ 6.73(d, 1H, J=10.1Hz), δ 14.50(s, 1H)
¹³C-NMR(CDCl₃); δ 8.45, δ 13.98, δ 23.30, δ 28.24, δ 38.33, δ 39.02, δ 79.64, δ 102.73, δ 104.24, δ 105.99, δ 110.45, δ 115.94, δ 126.39, δ 156.53, δ 157.46, δ 158.53, δ 159.41, δ 162.92, δ 206.94
MS(EI); 343(5.1, M+1), 342(24.9, M+), 327(100), 313(7.2), 299(9.0), 285(5.6)
IR(KBr); 2978, 2939, 1730, 1610, 1557, 1379 cm⁻¹

### Example 11: 5-hydroxy-2,2-dimethyl-6-propionyl-10-propyl-2H,8H-benzo[1,2-b:3,4-b']dipyran-8-one (XII) [Step (i) in the presence of acid catalyst]

In a flask equipped with a Soxhlet extractor (with molecular sieves 4A) and a reflux condenser were charged 5,7-dihydroxy-8-propionyl-4-propylcoumarin (XI) (100 mg, 0.36 mmol), 3-methyl-2-butenal (100 mg, 1.19 mmol), p-toluenesulfonic acid monohydrate (10 mg, 0.05 mmol) and toluene (10 ml) and the mixture was heated under reflux for 2 hrs. After evaporation of toluene, the residue was purified by silica gel chromatography to give the title compound as a pale-yellow solid (66 mg, yield 50%).

### Example 12: (±)-12-oxocalanolide A (VII) [Step (j)]

Acetaldehyde diethyl acetal (0.015 ml, 0.11 mmol), trifluoroacetic acid (0.18 ml) and pyridine (0.09 ml) were added to 5-hydroxy-2,2-dimethyl-,6-propionyl-10-propyl-2H,8H-benzo[1,2-b:3,4-b']dipyran-8-one (XII) (20 mg, 0.06 mmol) and the mixture was heated at 140°C for 2.5 hrs. Acetaldehyde diethyl acetal (0.02 ml, 0.14 mmol) was further added and the mixture was heated at the same temperature for 2.5 hrs. The reaction mixture was cooled to room temperature, diluted with ethyl acetate and washed 3 times with a 10% NaHCO₃ aqueous solution. The organic layer was separated, and after drying, the solvent was evaporated and the residue was purified by silica gel chromatography to give the title compound (2 mg, yield 9%).

### Example 13::5-hydroxy-2,2-dimethyl-6-{(2-methyl)-2-butenoyl}-10-propyl-2H,8H-benzo[1,2-b:3,4-b']dipyran-8-one (XIV) [Step (k)]

(±)-10,11-cis-Dihydro-6,6,10,11-tetramethyl-4-propyl-2H,6H,12H-benzo[1,2-b:3,4-b':5,6-b'']tripyran-2,12-dione (VII') (130 mg, 0.35 mmol) was dissolved in tetrahydrofuran (2 ml) and a solution of sodium hydroxide (30 mg, 0.75 mmol) dissolved in water (0.3 ml) was added with vigorous stirring. The mixture was stirred at room temperature for 3.5 hrs. Water and IN hydrochloric acid were added to adjust the mixture to pH 3, and the mixture was extracted with ethyl acetate. After drying over magnesium sulfate, the solvent was evaporated, and the residue was purified by silica gel chromatography to give the title compound as a pale-yellow solid (25 mg, yield 19%). mp : 110 - 112°C
¹H-NMR(CDCl₃); δ 1.05(t, 3H, J=7.3Hz), δ 1.54(s, 6H) , δ 1.67(sextet, 2H, J=7.5Hz), δ 1.84(dd, 3H, J=0.9Hz, 7.0Hz), δ 1.98(s, 3H), δ 2.89(t, 2H, J=7.7Hz), δ 5.59(d, 1H, J=10.0Hz), δ 5.96(s, 1H), δ 6.16(dq, 1H, J=1.3Hz, 6.9Hz), δ 6.72(d, 1H, J=10.0Hz),δ 12.03(s, 1H)
¹³C-NMR(CDCl₃), δ 13.16, δ 14.00, δ 14.33, δ 23.21, δ 28.26, δ 38.79, δ 79.41, δ 102.91, δ 104.11, δ 105.92, δ 110.92, δ 115.95, δ 126.56, δ 134.58, δ 139.42, δ 155.86, δ 155.99, δ 157.92, δ 159.28, δ 160.35, δ 200.92
MS(EI); 369(4.9, M+1), 368(19.1, M+), 354(23.1), 353(100), 325(9.8), 297(7.3), 269(4.2)
IR(KBr); 2957, 1736, 1608, 1578, 1366, 1275 cm⁻¹

### Example 14: 5-hydroxy-2,2-dimethyl-6-{(2-methyl)-2-butenoyl}-10-propyl-2H,8H-benzo[1,2-b:3,4-b']dipyran-8-one (XIV) [Step (k)]

A mixture (163 mg, 0.44 mmol) containing (±)-12-oxocalanolide A (VII) and (±)-10,11-cis-dihydro-6,6,10,11-tetramethyl-4-propyl-2H,6H,12H-benzo[1,2-b:3,4-b':5,6-b'']tripyran-2,12-dione (VII') at a ratio of 1.00:1.98 was dissolved in tetrahydrofuran (1 ml), and a solution of sodium hydroxide (35 mg, 0.88 mmol) dissolved in water (0.35 ml) was added with vigorous stirring. The mixture was stirred at room temperature for 3 hrs. Water and 1N hydrochloric acid were added to adjust the mixture to pH 4, and the mixture was extracted with ethyl acetate. After drying over magnesium sulfate, the solvent was evaporated and the residue was purified by silica gel chromatography to give the title compound as a pale-yellow solid (34 mg, yield 21%).

### Example 15: (±)-12-oxocalanolide A (VII) [Step (1)]

5-Hydroxy-2,2-dimethyl-6-{(2-methyl)-2-butenoyl}-10-propyl-2H, 8H-benzo[1,2-b:3,4-b']dipyran-8-one (XIV) (25 mg, 0.07 mmol) and triethylamine (10 ul, 0.07 mmol)were dissolved in tetrahydrofuran (0.5 ml) and the mixture was stirred at room temperature for 16 hrs. The reaction mixture was purified by silica gel chromatography to give the title compound as a white solid (14 mg, yield 57%).

### Example 16: (±)-12-oxocalanolide A (VII) [Step (m)]

(±)-10,11-cis-Dihydro-6, 6,10, 11-tetramethyl-4-propyl-2H,6H,12H-benzo[1,2-b:3,4-b':5,6-b"]tripyran-2,12-dione (VII') (35 mg, 0.10 mmol) was dissolved in toluene (1 ml) and conc. sulfuric acid (16 mg, 0.16 mmol) was added. The mixture was heated at 110°C for 2 hrs. Water was added to the reaction mixture, and the mixture was extracted with ethyl acetate. After drying over magnesium sulfate, the solvent was evaporated and the residue was purified by silica gel chromatography to give the title compound as a white solid (16 mg, yield 44%).

### Industrial Applicability

As is clear from the foregoing description, the present invention can provide a more convenient and industrially practical method for the synthesis of Calanolide A from an easily available starting material.

This application is based on a patent application No. 391248/2000 filed in Japan, the contents of which are hereby incorporated by reference.

## Claims

1. A production method of Calanolide A, which comprises
(a) a step of reacting phloroglucinol (I) 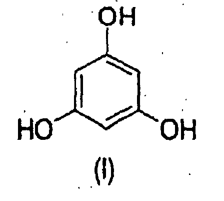 or a hydrate thereof, with a β-ketoester represented by the formula (II) 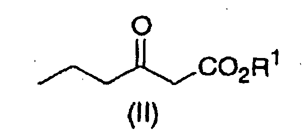 wherein R¹ is an alkyl group optionally having substituent(s), a cycloalkyl group optionally having substituent(s) or an aryl group optionally having substituent(s), in the presence of an acid catalyst to give 5,7-dihydroxy-4-propylcoumarin (III) 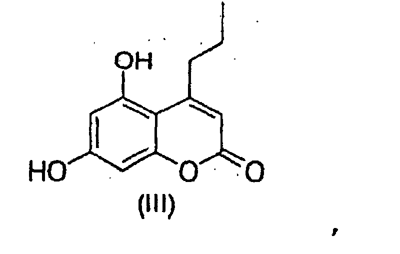
(b) a step of reacting 5,7-dihydroxy-4-propylcoumarin (III) with an acylating agent represented by the formula (IV) 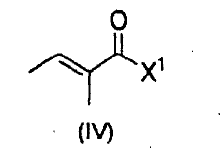 wherein X¹ is an alkoxyl group optionally having substituent(s), a cycloalkyloxy group optionally having substituent(s), an aryloxy group optionally having substituent(s), an acyloxy group optionally having substituent(s), an alkoxycarbonyloxy group optionally having substituent(s), a substituted amino group, a substituted sulfonyloxy group, a substituted phosphonyloxy group or a halogen atom to give 5-hydroxy-7-{(2-methyl)-2-butenoyloxy}-4-propylcoumarin (V) 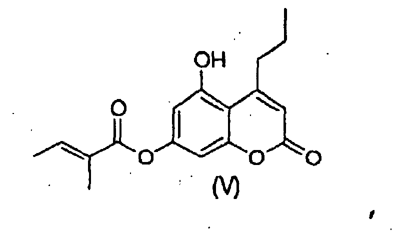
(c) a step of subjecting 5-hydroxy-7-{(2-methyl)-2-butenoyloxy}-4-propylcoumarin (V) to an intramolecular rearrangement reaction to give 5,7-dihydroxy-8-{(2-methyl)-2-butenoyl}-4-propylcoumarin (VI) 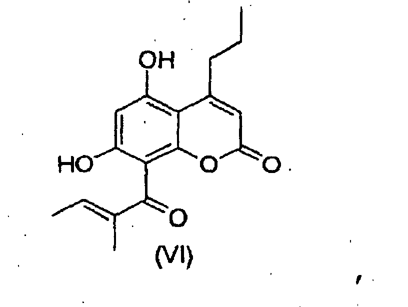
(d) a step of reacting 5,7-dihydroxy-8-{(2-methyl)-2-butenoyl}-4-propylcoumarin (VI) with 3-methyl-2-butenal to give 12-oxocalanolide A (VII) 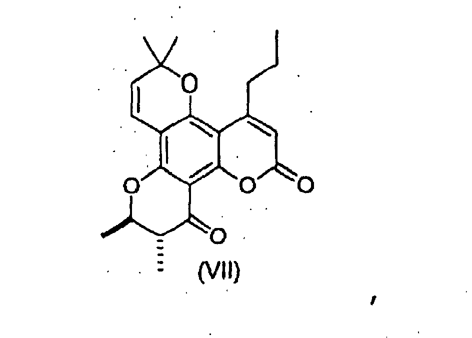
(e) a step of reducing 12-oxocalanolide A (VII) to give Calanolide A (VIII).

2. The method according to claim 1, wherein a Lewis acid catalyst is used in Step (b).

3. The method according to claim 1, wherein a Lewis acid catalyst is used in Step (c).

4. The method according to claim 1, wherein an acid catalyst is used in Step (d).

5. The method according to claim 1, wherein a base catalyst is used in Step (d).

6. The method according to claim 5, wherein the base catalyst is a tertiary amine.

7. The method according to claim 1, wherein the reduction in Step (e) is carried out using lithium aluminum hydride, lithium bis(2-methoxyethoxy)aluminum hydride, lithium tri-tert-butoxyaluminum hydride, sodium borohydride or lithium borohydride.

8. 5-Hydroxy-7-{(2-methyl)-2-butenoyloxy}-4-propylcoumarin (V)

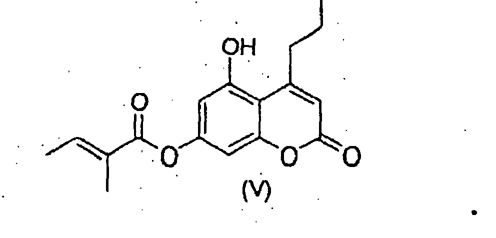

9. A production method of 5-hydroxy-7-{(2-methyl)-2-butenoyloxy}-4-propylcoumarin (V) 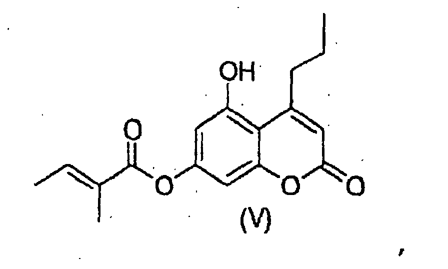 which comprises reacting 5,7-dihydroxy-4-propylcoumarin (III) 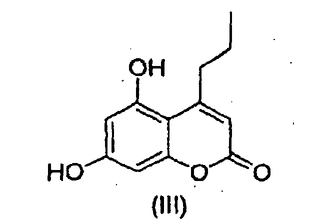 with an acylating agent represented by the formula (IV) 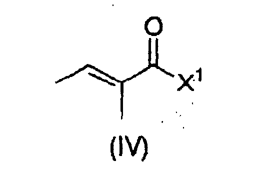 wherein X¹ is an alkoxyl group optionally having substituent(s), a cycloalkyloxy group optionally having substituent(s), an aryloxy group optionally having substituent(s), an acyloxy group optionally having substituent(s), an alkoxycarbonyloxy group optionally having substituent(s), a substituted amino group, a substituted sulfonyloxy group, a substituted phosphonyloxy group or a halogen atom.

10. A production method of 5,7-dihydroxy-8-{(2-methyl)-2-butenoyl}-4-propylcoumarin (VI) 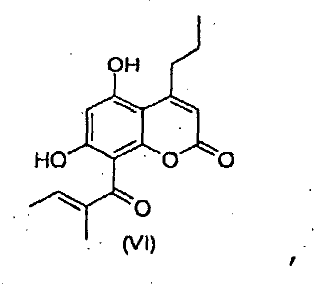 which comprises subjecting 5-hydroxy-7-{(2-methyl)-2-butenoyloxy}-4-propylcoumarin (V) 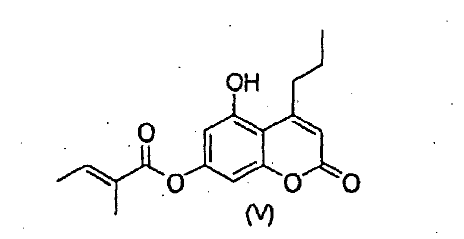 to an intramolecular rearrangement reaction.

11. A production method of 12-oxocalanolide A (VII) 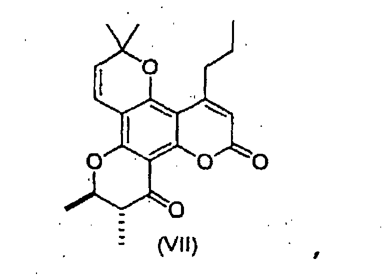 which comprises reacting 5,7-dihydroxy-8-{(2-methyl)-2-butenoyl}-4-propylcoumarin (VI) 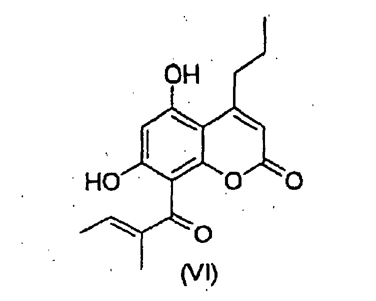 with 3-methyl-2-butenal.

12. The method according to claim 11, wherein an acid catalyst is used.

13. The method according to claim 11, wherein a base catalyst is used.

14. The method according to claim 13, wherein the base catalyst is a tertiary amine

15. A production method of 12-oxocalanolide A (VII) 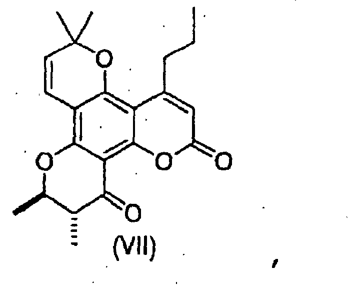 which comprises reacting 2, 3-trans-dihydro-6-hydroxy-2,3-dimethyl-7-propyl-lH,9H-benzo[ 1,2-b:3,4-b']dipyran-1,9-dione (IX) 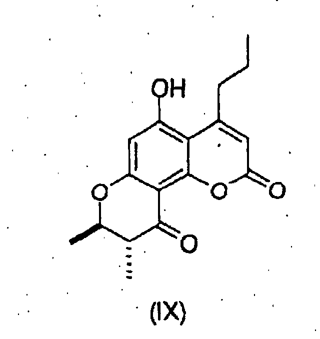 with 3-methyl-2-butenal.

16. The method according to claim 15, wherein an acid catalyst is used.

17. The method according to claim 15, wherein a base catalyst is used.

18. The method according to claim 17, wherein the base catalyst is a tertiary amine.

19. A production method of Calanolide A, which comprises
(a) a step of reacting phloroglucinol (I) 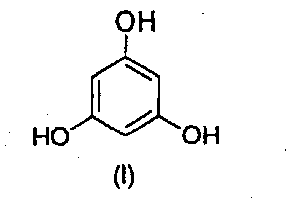 or a hydrate thereof with a β-ketoester represented by the formula (II) 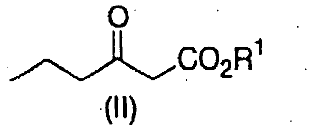 wherein R¹ is an alkyl group optionally having substituent(s), a cycloalkyl group optionally having substituent(s) or an aryl group optionally having substituent(s), in the presence of an acid catalyst to give 5,7-dihydroxy-4-propylcoumarin (III) 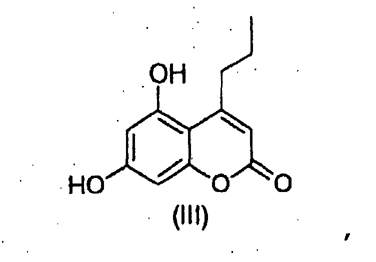
(h) a step of reacting 5,7-dihydroxy-4-propylcoumarin (III) with an acylating agent represented by the formula (X) 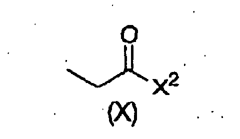 wherein X² is an alkoxyl group optionally having substituent(s), a cycloalkyloxy group optionally having substituent(s), an aryloxy group optionally having substituent(s), an acyloxy group optionally having substituent(s), an alkoxycarbonyloxy group optionally having substituent(s), a substituted amino group, a substituted sulfonyloxy group, a substituted phosphonyloxy group or a halogen atom, to give 5,7-dihydroxy-8-propionyl-4-propylcoumarin (XI) 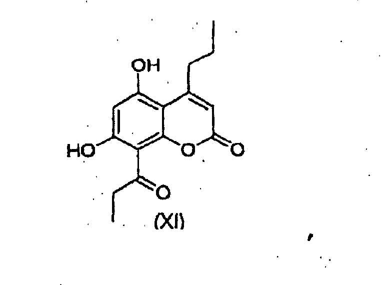
(i) a step of reacting 5,7-dihydroxy-8-propionyl-4-propylcoumarin (XI) with 3-methyl-2-butenal to give 5-hydroxy-2,2-dimethyl-6-propionyl-10-propyl-2H,8H-benzo[1,2-b:3,4-b']dipyran-8-one (XII) 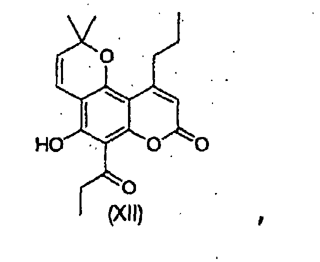
(j) a step of reacting 5-hydroxy-2,2-dimethyl-6-propionyl-10-propyl-2H,8H-benzo[1,2-b:3,4-b']-dipyran-8-one (XII) with an acetal of acetaldehyde represented by the formula (XIII) 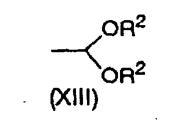 wherein two R² conveniently having the same superscript may be the same group or different groups, an alkyl group optionally having substituent(s), a cycloalkyl group optionally having substituent(s) or an aryl group optionally having substituent(s), or two R² may be joined to form a ring structure, in the presence of an acid catalyst to give 12-oxocalanolide A (VII) 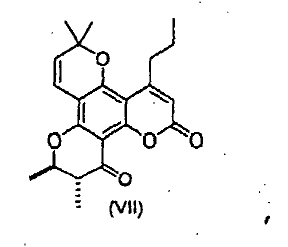 and
(e) a step of reducing 12-oxocalanolide A (VII) to give Calanolide A (VIII)

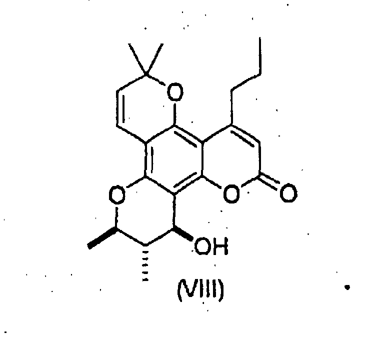

20. The method according to claim 19, wherein a Lewis acid catalyst is used in Step (h).

21. The method according to claim 19, wherein an acid catalyst is used in Step (i).

22. The method according to claim 19, wherein a base catalyst is used in Step (i).

23. The method according to claim 22, wherein the base catalyst is a tertiary amine.

24. The method according to claim 19, wherein the reduction in Step (e) is carried out using lithium aluminum hydride, lithium bis(2-methoxyethoxy)aluminum hydride, lithium tri-tert-butoxyaluminum hydride, sodium borohydride or lithium borohydride.

25. A production method of 5-hydroxy-2,2-dimethyl-6-propionyl-10-propyl-2H,8H-benzo[1,2-b:3,4-b']dipyran-8-one (XII) 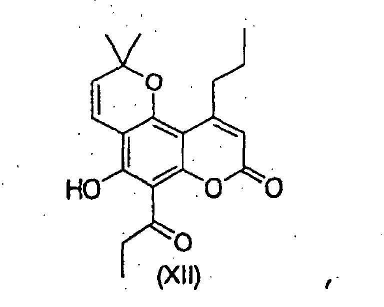 which comprises reacting 5,7-dihydrpxy-8-propionyl-4-propylcoumarin (XI) 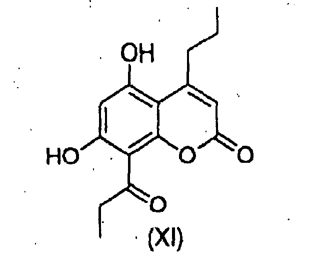 with 3-methyl-2-butenal.

26. The method according to claim 25, wherein an acid catalysis used.

27. The method according to claim 25, wherein a base catalyst is used.

28. The method according to claim 27, wherein the base catalyst is a tertiary amine.

29. A production method of 12-oxocalanolide A, which comprise; (k) a step of obtaining 5-hydroxy-2,2-dimethyl-6-{(2-methyl)-2-butenoyl}-10-propyl-2H,8H-benzo[1,2-b:3,4-b']-dipyran-8-one (XIV) 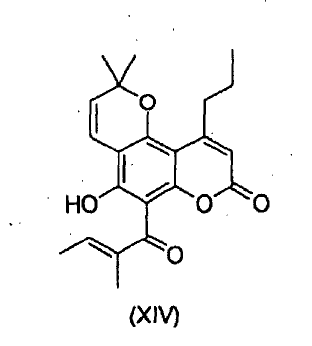 from 10,11-cis-dihydro-6,6,10,11-tetramethyl-4-propyl-2H, 6H, 12H-benzo[1,2-b:3,4-b':5,6-b'']tripyran-2,12-dione (VII') 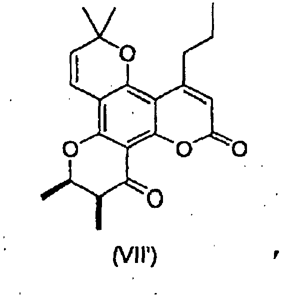 and
(1) a step of subjecting 5-hydroxy-2,2-dimethyl-6-{(2-methyl)-2-butenoyl}-10-propyl-2H,8H-benzo[1,2-b:3,4-b']dipyran-8-one (XIV) to a intramolecular cyclization reaction to give 12-oxocalanolide A (VII)

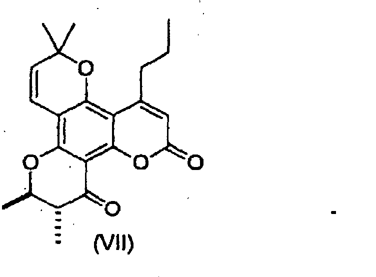

30. The method according to claim 29, wherein a base is used in Step (k).

31. A production method of 5-hydroxy-2,2-dimethyl-6-{(2-methyl)-2-butenoyl}-10-propyl-2H,8H-benzo[1,2-b:3,4-b']dipyran-8-one (XIV) 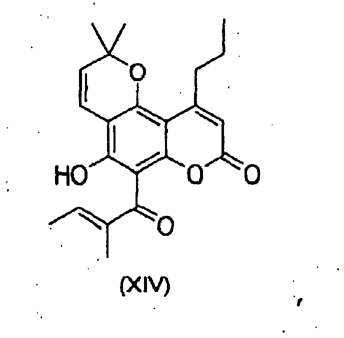 which comprises treating 10, 11-dihydro-6,'6,10,11-tetramethyl-4-propyl-2H,6H,12H-benzo[1,2-b:3,4-b':5,6-b'')tripyran-2,12-dione (VII'') 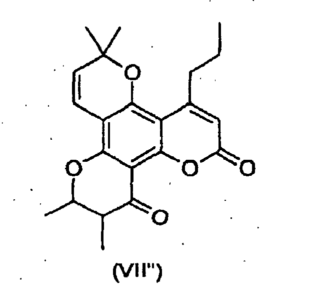 with a base.

32. A production method of 12-oxocalanolide A (VII) 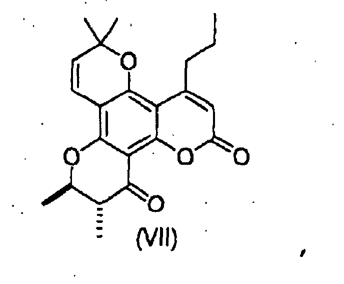 which comprises reacting an acid with 10,11-cis-dihydro-6,6,10,11-tetramethyl-4-propyl-2H,6H,12H-benzo[1,2-b:3,4-b':5, 6-b"]tripyran-2,12-dione (VII')
